# EUROPEAN PATENT SPECIFICATION

(11) **EP 3 880 155 B1**
(45) Date of publication and mention of the grant of the patent: **27.12.2023**
(21) Application number: 19802175.0
(22) Date of filing: 13.11.2019
(51) Int. Cl.: A61K 6/79, A61K 6/887, A61K 6/90

(54) **BIFUNCTIONAL AND POLYFUNCTIONAL COINITIATORS IN DENTAL COMPOSITIONS**
BIFUNKTIONELLE UND POLYFUNKTIONELLE CO-INITIATOREN IN ZAHNÄRZTLICHEN ZUSAMMENSETZUNGEN
COINITIATEURS BIFONCTIONNELS ET POLYFONCTIONNELS DANS DES COMPOSITIONS DENTAIRES

(30) Priority: 15.11.2018 EP 18206588
(43) Date of publication of application: 22.09.2021
(73) Proprietor: DENTSPLY DETREY GmbH, 78467 Konstanz (DE)
(72) Inventor: KLEE, Joachim, 78315 Radolfzell (DE); ELSNER, Oliver, 78315 Radolfzell (DE); SZILLAT, Florian, 78462 Konstanz (DE); RENN, Caroline, 78224 Singen (DE)
(74) Representative: Hartz, Nikolai
(86) International application number: PCT/EP2019/081241
(87) International publication number: WO 2020/099518

(56) References cited:
- EP-A1- 2 033 949
- WO-A1-2015/124559
- WO-A1-2016/149488
- GE XUEPING ET AL: "Synthesis and Evaluation of a Novel Co-Initiator for Dentin Adhesives: Polymerization Kinetics and Leachables Study", JOM: JOURNAL OF METALS, SPRINGER NEW YORK LLC, UNITED STATES, vol. 67, no. 4, 27 February 2015 (2015-02-27), pages 796-803, XP035474709, ISSN: 1047-4838, DOI: 10.1007/S11837-015-1335-6 [retrieved on 2015-02-27]

## Description

### Field of the invention

The present invention relates to the use of a specific coinitiator for the preparation of a crosslinked dental composition.

### Background of the invention

Curable dental compositions containing free-radically polymerizable resins are known and commonly comprise a polymerizable monomer, a crosslinker, and an initiator system. The initiator system may be a photoinitiator system, a redox initiator system or a dual cure initiator system.

Photoinitiator systems contain a sensitizer in combination with a coinitiator. Camphor quinone in combination with a tertiary amine is frequently used as dental photoinitiator system.

Redox initiator systems contain an oxidizing agent and a reducing agent as essential components. Aromatic tertiary amines, ascorbic acid and thiourea derivatives are frequently used as reducing agents in dental compositions.

A dual cure initiator system combines a photoinitiator system and a redox initiator system.

Radical initiator systems need to fulfil specific requirements in order to be useful in dental compositions. For example, dental initiator systems need to be miscible and storage stable when incorporated into a dental composition, and at the same time provide a high polymerization efficiency including a high conversion, good curing rate and depth of cure when activated for providing a useful direct dental restoration.

In a dental initiator system, aromatic amines are often preferred coinitiators for reasons of stability and activity. For example, ethyl-4-dimethylaminobenzoate (DMABE) is a popular coinitiator in dental compositions. Moreover, US 10,058,487 B2 discloses specific tertiary amines which can be used for the preparation of an improved dental material for direct tooth restoration.

WO 2015/124559 discloses the use of a tertiary aromatic amine having a benzene ring to which at least one dialkylamine group and at least one further group are directly bonded, the other group being selected from: i. carboxylic acid ester groups containing at least one polyoxyalkylene group having at least 2 oxyethylene and / or oxypropylene units, and ii. amide groups as a coinitiator in a polymerizable dental material. EP 2 033 949 A1 discloses radiation-curable compositison for inkjet printing comprising a free radical polymerisable monomer and a photoinitiator system. The photoinitiator system comprises at least one polymerisable aromatic tertiary amine of formula (I) as co-initiator. The use of these reduces the amount of extractables, i.e. reduces leaching of the tertiary amines.

However, tertiary amines for use as coinitiators in dental compositions may leach out of the cured composition which may give rise to toxicological concerns, in particular in the case of aromatic amine compounds.

### Summary of the invention

It is the problem of the present invention to provide a dental composition comprising a polymerizable monomer and an initiator system comprising an tertiary aromatic amine coinitiator, which composition provides good storage stability and a high polymerization efficiency including a high conversion, good curing rate and depth of cure, and which composition has significantly reduced, preferably no leaching problems of the tertiary aromatic amines.

Moreover, it is the problem of the present invention to provide a use of a specific coinitiator for the preparation of a dental composition.

The present invention provides the use of coinitiator, as defined in the appended claims.

The present invention is based on the recognition that a dental composition comprising a coinitiator compound of formula (I) provides improved polymerization efficiency including a high conversion, good curing rate and depth of cure, and does not pose leaching problems of tertiary aromatic amines. According to the present invention, a compound of formula (I) acts as a chain extender or is integrated as a crosslinking site into the polymer network during the curing reaction of the dental composition so that leaching of an aromatic amine is excluded while at the same time a high polymerization efficiency and storage stability are provided.

A coinitiator compound of formula (I) according to the present invention is adapted to act as a crosslinker either by initiating polymerization of at least two polymer chains or by initiating polymerization of a polymer chain, and taking part in the polymerization of a copolymerizable compound having a polymerizable carbon-carbon double bond. Accordingly, the compound of formula (I) will be incorporated into the polymer network either by initiating a polymerization reaction, or by copolymerization with the copolymerizable monomer. Therefore, leaching problems are avoided. Moreover, a dental composition of the present invention provides good storage stability when incorporated into a dental composition, but at the same time provides a high polymerization efficiency including a high conversion, good curing rate and depth of cure when activated.

### Detailed description of preferred embodiments

The terms "polymerization" or "polymerizable" relate to the combining by covalent bonding of many smaller molecules, such as monomers, oligomers or polymers in the form of radically polymerizable monomers, to form larger molecules, i.e. macromolecules or polymers. The monomers may be combined to form linear macromolecules or three-dimensional macromolecules, commonly referred to as crosslinked polymers.

The term "radically polymerizable" as used herein in connection with monomers (a) means any monomer capable of radical polymerization. Typically, monomers (a) are radically polymerizable due to a polymerizable double bond, preferably one or more carbon-carbon double bonds. Examples of the polymerizable double bond include vinyl, conjugated vinyl, allyl, acryl, methacryl and styryl. More preferably, the polymerizable double bound is selected from the group consisting of acryl, methacryl and styryl. Acryl and methacryl may be (meth)acryloyl or (meth)acrylamide. Most preferably, for monomers (a), the polymerizable double bound is acryl or methacryl.

The term "initiator system" means any system comprising (b-1) a sensitizer or an oxidizing agent capable of oxidizing a reducing agent of a redox initiator system, and a coinitiator (b-2), forming free radicals when activated, e. g. by thermal energy, or by exposure to light and/or interaction with one or more further compounds in a photochemical reaction or redox process, whereby polymerization of polymerizable monomers is initiated. The term "initiator system" refers to any photoinitiator system, or redox initiator system (b-1) and (b-2).

The initiator system may be a photoinitiator system, wherein the term "photoinitiator system" as used herein relates to a system consisting of a sensitizer (b-1) and a coinitiator (b-2) and optionally one or more further initiator compounds, wherein the sensitizer, coinitiator and other further initiator compounds are capable of generating alone or in combination free radicals when irradiated with light having a wavelength in the range of from 400 to 800 nm.

Alternatively, the initiator system may be a redox initiator system and the term "redox initiator system" as used herein means a system comprising a combination of (b-1) an oxidizing agent capable of oxidizing a reducing agent of a redox initiator system, and a coinitiator (b-2) acting as a reducing agent, and optionally a catalyst such as a metal salt. The redox initiator system provides for a redox reaction in which radicals are formed. These radicals initiate polymerisation of a radically polymerizable monomer. Typically, a redox initiator system is activated, that is redox reaction is initiated, by bringing the redox initiator system in contact with water and/or an organic solvent providing for at least partial dissolution of the oxidising agent and the reducing agent. The optional catalyst may be added to accelerate the redox reaction and thus the polymerization of the radically polymerizable monomer.

The term "coinitiator" refers to a molecule that produces a chemical change in another molecule, whereby a polymerization is initiated.

The term "sensitizer" refers to a molecule that produces a chemical change in another molecule such as the coinitiator in a photochemical process. Preferably, the initiator system comprises a sensitizer having an absorption maximum in the range of from 400 to 800 nm.

The term "curing" means the polymerization of functional polymerizable monomers such as monomers, oligomers or polymers, into a polymer network, preferably a crosslinked polymer network.

The term "curable" refers to dental composition that will radically polymerize upon initiation when (co)polymerizable monomers, oligomers and polymerizable polymers are polymerized.

The term "peroxides" as used herein means compounds of the formula Rₓ-O-O-R_{y} in which Rₓ and R_{y} independently from each other denote any suitable organic or inorganic group. For example, "organic peroxides" may include peroxyesters, such as tert-butylperoxybenzoate, in which one organic group Rₓ or R_{y} is an acyl group. An acyl group is an organic group having a carbonyl group (-(C=O)-) to which, in the present case, the peroxo moiety (-O-O-) is bound. Furthermore, "organic peroxides" may also include di-acylperoxides, such as benzoyl peroxide, in which both organic residues Rₓ and R_{y} represent acyl groups, dialkylperoxides, such as as di-tert-butyl peroxide, in which both organic residues Rₓ and R_{y} are alkyl residues, and peroxydicarbonates, such as diisopropyl peroxydicarbonate, in which both organic residues Rₓ and R_{y} represent carbonyloxyalkyl groups. For example, "inorganic peroxides" may include potassium persulfate or potassium peroxidisulfate.

The term "hydroperoxides" as used herein means compounds of formula Rₓ-O-O-H in which Rₓ denotes any organic group. For example, "organic hydroperoxides" may include peroxy acids, such as peroxybenzoic acid, in which the organic group Rₓ is an acyl group.

The term "polymerization accelerator" means any substance which is able to increase the reactivity of the redox initiator system. Examples of the polymerization accelerator include sulfinic acids, sulfinates, sulfites, hydrogen sulfites, aldehydes, thiourea compounds, barbituric acid derivatives, triazine compounds, halogen compounds, and thiol compounds.

The present invention relates to a dental composition. The dental composition may be a dental material to be used in the oral cavity. Preferably, the dental composition according to the invention is selected from a dental adhesive composition, a dental bonding agent, a dental primer, a dental impression material, a dental infiltrant, a dental desensitizing composition, a pulp capping composition, a dental composite, a dental cement, a dental glass ionomer cement, a dental resin-modified dental cement, a dental sealer, a pit and fissure sealant, a seal and protecting composition for naked tooth necks, and a dental root canal sealer composition.

Preferably, the dental composition is selected a dental bonding agent, a dental sealer, a dental composite, a resin-modified dental cement, and a dental impression material.

### The polymerizable monomer (a)

The dental composition of the present invention comprises (a) a polymerizable monomer. The dental composition may comprise one or more polymerizable monomers (a).

The polymerizable monomer (a) is a radically polymerizable monomer, oligomer or polymer and is not particularly limited concerning its radically polymerizable groups. The polymerizable monomer (a) may have one or more radically polymerizable groups. At least one radically polymerizable group may for example be a radically polymerizable carbon-carbon double bond, which may be selected from a (meth)acryloyl group, a (meth)acrylamide group, or an allyl group.

Suitable examples for a polymerizable monomer (a) in the form of a monomer may be selected from the group consisting of (meth)acrylates, amides of acrylic or methacrylic acid, urethane acrylates or methacrylates, and polyol acrylates or methacrylates.

(Meth)acrylates may be preferably selected from compounds of the following formulae (A), (B) and (C): 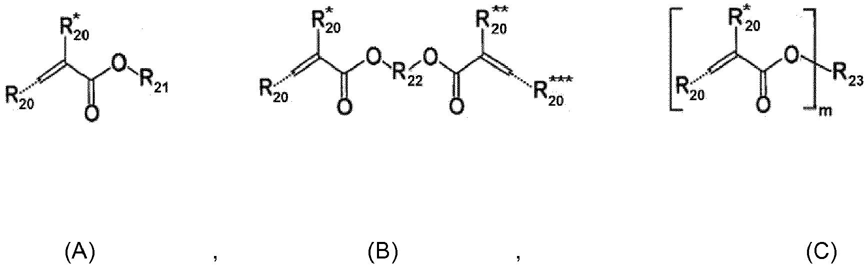
wherein R₂₀, R*₂₀, R**₂₀, R***₂₀ independently represent a hydrogen atom, -COOM, a linear C₁₋₁₈ or branched C₃₋₁₈ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M*, a C₃ to C₁₈ cycloalkyl group which may be substituted by a C₁₋₁₆ alkyl group, a C₆₋₁₄ aryl or C₃₋₁₄ heteroaryl group, or a C₅ to C₁₈ aryl or C₃ to C₁₈ heteroaryl group, -COOM, -PO₃M, -O-PO₃M₂ or -SO₃M*,
R₂₁ represents a hydrogen atom, a linear C₁₋₁₈ or branched C₃₋₁₈ alkyl group or C₂ to C₁₈ alkenyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M*, a C₃ to C₁₈ cycloalkyl group which may be substituted by a C₁₋₁₆ alkyl group, a C₆₋₁₄ aryl or C₃₋₁₄ heteroaryl group, - COOM, -PO₃M, -O-PO₃M₂ or-SOsM*, or a C₅ to C₁₈ aryl or C₃ to C₁₈ heteroaryl group,
R₂₂ represents a divalent organic residue having from 1 to 45 carbon atoms, whereby the divalent organic residue may contain at least one of from 1 to 7 C₃₋₁₂ cycloalkylene group(s), 1 to 7 C₆₋₁₄ arylene groups, 1 to 7 carbonyl groups, 1 to 7 carboxyl groups (-(C=O)-O- or -O-(C=O-), 1 to 7 amide groups (-(C=O)-NH- or -NH-(C=O)-) or 1 to 7 urethane groups (-NH-(C=O)-O- or-O-(C=O)-NH-), and 1 to 14 heteroatoms selected from oxygen, nitrogen and sulphur, which divalent organic residue may be substituted with one or more substituents selected from the group consisting of a hydroxyl group, a thiol group, a C₆₋₁₄ aryl group, -COOM, -PO₃M, -O-PO₃M₂ or -SO₃M*; preferably R₂₂ is a C₁ to C₁₈ alkylene group or a C₂ to C₁₈ alkenylene group, which may be substituted by one or more -OH group(s), which alkylene or alkenylene group may contain at least one of 1 to 4 C₆₋₁₀ arylene groups, 1 to 4 urethane groups (-NH-(C=O)-O- or -O-(C=O)-NH-), and 1 to 8 oxygen atoms;
R₂₃ represents a saturated di- or multivalent substituted or unsubstituted C₂ to C₁₈ hydrocarbon group, a saturated di- or multivalent substituted or unsubstituted cyclic C₃ to C₁₈ hydrocarbon group, a di- or multivalent substituted or unsubstituted C₄ to C₁₈ aryl or heteroaryl group, a di- or multivalent substituted or unsubstituted C₅ to C₁₈ alkylaryl or alkylheteroaryl group, a di- or multivalent substituted or unsubstituted C₇ to C₃₀ aralkyl group, or a di- or multivalent substituted or unsubstituted C₂ to C₄₅ mono-, di-, or polyether residue having from 1 to 14 oxygen atoms, and
m is an integer, preferably in the range from 1 to 10,
wherein M of any one of R₂₀, R*₂₀, R**₂₀, R***₂₀, R₂₁, and R₂₂, which M are independent from each other, each represent a hydrogen atom or a metal atom, and
M* of any one of R₂₀, R*₂₀, R**₂₀, R***₂₀, R₂₁, and R₂₂, which M are independent from each other, each represent a metal atom.

For R₂₀, R*₂₀, R**₂₀ and R***₂₀, the linear C₁₋₁₈ or branched C₃₋₁₈ alkyl group may e.g. be methyl, ethyl, n-propyl, i-propyl, n-butyl, isobutyl, tert-butyl, sec-butyl, pentyl or hexyl. For R₂₁ and R*₂₁, the C₁₋₁₈ alkyl group or C₂₋₁₈ alkenyl group may e.g. be eth(en)yl, n-prop(en)yl, i-prop(en)yl , n-but(en)yl, isobut(en)yl, tert-but(en)yl sec-but(en)yl, pent(en)yl or hex(en)yl.

For R₂₀, R*₂₀, R**₂₀, R***₂₀ and R₂₁ an aryl group may, for example, be a phenyl group or a naphthyl group, and a C₃₋₁₄ heteroaryl group may contain 1 to 3 heteroatoms selected from nitrogen, oxygen and sulfur.

For R₂₂, in the phrase "divalent organic residue may contain at least one of' means that the groups which may be contained in the divalent organic residue are incorporated in the divalent organic residue by means of covalent bonding. For example, in BisGMA, two aryl groups in the form of phenyl and two heteroatoms in the form of oxygen are incorporated into the divalent organic residue of R₂₂. Or, as a further example, in UDMA, two urethane groups (-NH-(C=O)-O- or -O-(C=O)-NH-) are incorporated in the divalent organic residue of R₂₂.

In formula (B), the dotted bond indicates that R₂₀ and R***₂₀ may be in (Z) or (E) configuration relative to CO.

Preferably, in formulae (A), (B) and (C), R₂₀, R*₂₀, R**₂₀ and R***₂₀ independently represent a hydrogen atom, a linear C₁₋₁₆ or branched C₃₋₁₆ alkyl group which may be substituted by a C₃₋₆ cycloalkyl group, a C₆₋₁₄ aryl or C₃₋₁₄ heteroaryl group, a C₃₋₆ cycloalkyl group which may be substituted by a C₁₋₁₆ alkyl group, a C₆₋₁₄ aryl or C₃₋₁₄ heteroaryl group, a C₆₋₁₄ aryl or C₃₋₁₄ heteroaryl group. More preferably, in formula (B), R₂₀, R*₂₀, R**₂₀ and R***₂₀ independently represent a hydrogen atom, a linear C₁₋₈ or branched C₃₋₈ alkyl group which may be substituted by a C₄₋₆ cycloalkyl group, a C₆₋₁₀ aryl or C₄₋₁₀ heteroaryl group, a C₄₋₆ cycloalkyl group which may be substituted by a C₁₋₆ alkyl group, a C₆₋₁₀ aryl or C₄₋₁₀ heteroaryl group or a C₆₋₁₀ aryl group. Even more preferably, R₂₀, R*₂₀, R**₂₀ and R***₂₀ independently represent a hydrogen atom, a linear C₁₄ or branched C₃ or C₄ alkyl group which may be substituted by a cyclohexyl group or a phenyl group, or a cyclohexyl group which may be substituted by a C₁₋₄ alkyl group. Most preferably, R₂₀, R*₂₀, R**₂₀ and R***₂₀ independently represent a hydrogen atom or a linear C₁₋₄ or branched C₃ or C₄ alkyl group.

Preferably, in formula (A), R₂₁ represents a hydrogen atom, a linear C₁₋₁₆ or branched C₃₋₁₆ alkyl group or C₂₋₁₆ alkenyl group which may be substituted by a C₃₋₆ cycloalkyl group, a C₆₋₁₄ aryl or C₃₋₁₄ heteroaryl group, a C₃₋₆ cycloalkyl group which may be substituted by a C₁₋₁₆ alkyl group, a C₆₋₁₄ aryl or C₃₋₁₄ heteroaryl group, a C₆₋₁₄ aryl or C₃₋₁₄ heteroaryl group. More preferably, R₂₁ represents a hydrogen atom, a linear C₁₋₁₀ or branched C₃₋₁₀ alkyl or C₂₋₁₀ alkenyl group group which may be substituted by a C₄₋₆ cycloalkyl group, a C₆₋₁₀ aryl or C₄₋₁₀ heteroaryl group, a C₄₋₆ cycloalkyl group which may be substituted by a C₁₋₆ alkyl group, a C₆₋₁₀ aryl or C₄₋₁₀ heteroaryl group or a C₆₋₁₀ aryl group. Even more preferably, R₂₁ represents is a hydrogen atom, a linear C₁₋₁₀ or branched C₃₋₁₀ alkyl group or linear C₂₋₁₀ or branched C₃₋₁₀ alkenyl group which may be substituted by a cyclohexyl group or a phenyl group, or a cyclohexyl group which may be substituted by a C₁₋₄ alkyl group. Yet even more preferably, R₂₁ represents an unsubstituted C₁₋₁₀ alkyl group or C₂₋₁₀ alkenyl group, still even more preferably an unsubstituted C₂₋₆ alkyl group or C₃₋₆ alkenyl group, and most preferably an ethyl group or an allyl group.

The (meth)acrylate compounds of formulae (A), (B) and (C) may be selected from the group consisting of methyl acrylate, methyl methacrylate, ethyl acrylate, ethyl methacry-late, propyl acrylate, propyl methacrylate, isopropyl acrylate, isopropyl methacrylate, 2-hydroxyethyl acrylate, 2-hydroxyethyl methacrylate (HEMA), hydroxypropyl acrylate, hydroxypropyl methacrylate, tetrahydrofurfuryl acrylate, tetrahydrofurfuryl methacrylate, glycidyl acrylate, glycidyl methacrylate, bisphenol A glycerolate dimethacrylat ("bisGMA", CAS-No. 1565-94-2), 4,4,6,16 (or 4,6,6,16)-tetramethyl-10,15-dioxo-11,14-dioxa-2,9-diazaheptadec-16-enoicacid 2-[(2-methyl-1-oxo-2-propen-1-yl)oxy]ethyl ester (CAS no. 72869-86-4)_(UDMA), glycerol mono-and di- acrylate such as 1,3-glycerol dimethacrylate (GDM), glycerol mono- and dimethacrylate, ethyleneglycol diacrylate, ethyleneglycol dimethacrylate, polyethyleneglycol diacrylate (where the number of repeating ethylene oxide units vary from 2 to 30), polyethyleneglycol dimethacrylate (where the number of repeating ethylene oxide units vary from 2 to 30 especially triethylene glycol dimethacrylate ("TEGDMA"), neopentyl glycol diacrylate, neopentylglycol dimethacrylate, trimethylolpropane triacrylate, trimethylol propane trimethacrylate, mono-, di-, tri-, and tetra- acrylates and methacrylates of pentaerythritol and dipentaerythritol, 1,3-butanediol diacrylate, 1,3-butanediol dimethacrylate, 1,4-butanedioldiacrylate, 1,4-butanediol dimethacrylate, 1,6-hexane diol diacrylate, 1,6-hexanediol dimethacrylate, di-2-methacry-loyloxethyl hexamethylene dicarbamate, di-2-methacryloyloxyethyl trimethylhex-anethylene dicarbamate, di-2-methacryloyl oxyethyl dimethylbenzene dicarbamate, methylene-bis-2-methacryloxyethyl-4-cyclohexyl carbamate, di-2-methacryloxyethyl-dimethylcyclohexane dicarbamate, methylene-bis-2-methacryloxyethyl-4-cyclohexyl carbamate, di-1-methyl-2-methacryloxyethyl-trimethyl-hexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-metha-cryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-1-chloromethyl-2-methacryloxyethyl4-cyclohexyl carbamate, 2,2'-bis(4-methacryloxyphenyl)propane, 2,2'bis(4-acryloxyphenyl)propane, 2,2'-bis[4(2-hydroxy-3-methacryloxy-phenyl)]propane, 2,2'-bis[4(2-hydroxy-3-acryloxy-phenyl)propane, 2,2'-bis(4-methacryloxy-ethoxyphenyl)propane, 2,2'-bis(4-acryloxyethoxyphenyl)propane, 2,2'-bis(4-methacryloxypropoxyphenyl)propane, 2,2'-bis(4-acryloxypropoxyphenyl)propane, 2,2'-bis(4-methacryloxydiethoxyphenyl)propane, 2,2'-bis(4-acryloxydiethoxyphenyl)propane, 2,2'-bis[3(4-phenoxy)-2-hydroxypropane-1-methacrylate]propane,and 2,2'-bis[3(4-phenoxy)-2-hydroxypropane-1-acrylate]propane.

Most preferably, a compound of formula (B) is selected from the group consisting of:

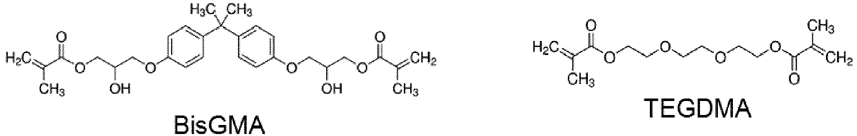

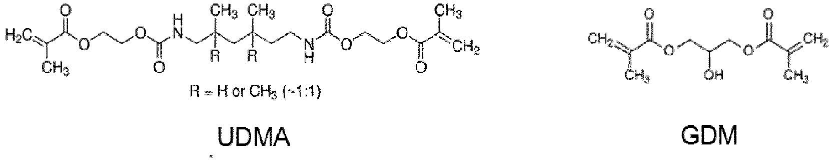

Particular preferred mono- or bis- or (meth)acrylamides and poly[(meth) acrylamides] have the following formulae (D), (E) and (F): 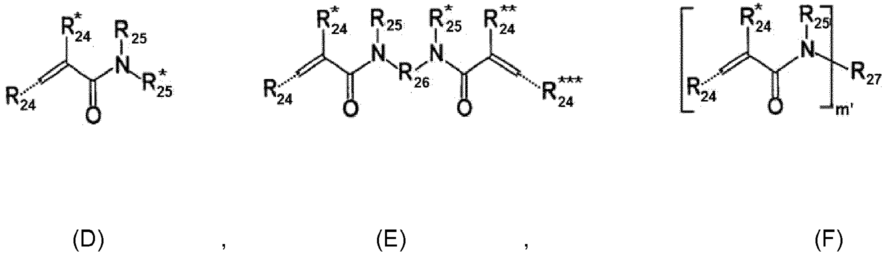 wherein R₂₄ R*₂₄, R**₂₄, R***₂₄ have the same meaning as R₂₀ R*₂₀, R**₂₀, R***₂₀ defined above for formulae (A), (B) and (C), R₂₅, R*₂₅ independently represent a residue having the same meaning as R₂₁ defined above for formula (A), and R₂₇ and m' have the same meaning as R₂₃ and m defined above for formula (C).

In formula (E), R₂₆ represents a divalent substituted or unsubstituted organic residue having from 1 to 45 carbon atoms, whereby said organic residue may contain at least one of 1 to 7 C₃₋₁₂ cycloalkylene group(s), 1 to 7 C₆₋₁₄ arylene groups, from 1 to 7 carbonyl groups, 1 to 7 carboxyl groups (-(C=O)-O- or -O-(C=O-), 1 to 7 amide groups (-(C=O)-NH- or-NH-(C=O)-), 1 to 7 urethane groups (-NH-(C=O)-O- or-O-(C=O)-NH-), and 1 to 14 heteroatoms selected from oxygen, nitrogen and sulphur, which divalent organic residue may be substituted with one or more substituent(s) selected from the group consisting of a hydroxyl group, a thiol group, a C₆₋₁₄ aryl group, -COOM, -PO₃M, -O-PO₃M₂ or-SO₃M* Preferably, R₂₆ is a C₁ to C₁₈ alkylene group or a C₂ to C₁₈ alkenylene group which may contain at least one of 1 to 4 C₆₋₁₀ arylene groups and C₃₋₈ cycloalkylene group, 1 to 4 urethane groups (-NH-(C=O)-O- or -O-(C=O)-NH-), and 1 to 8 oxygen atoms or nitrogen atoms.

For R₂₆, the phrase "divalent organic residue may contain at least one of ..." has an analogous meaning as defined above for R₂₂ of compound of formula (B).

In formulae (D), (E), (F), the dotted bond indicates that R₂₄ and R***₂₄ may be in (Z) or (E) configuration relative to CO.

In compound of formula (D), R₂₅ and R₂₅* may cooperatively form a ring in which R₂₅ and R₂₅* are linked by a C-C bond or a functional group selected from the group consisting of an ether group, a thioether group, an amine group and an amide group.

Preferred methacrylamides according to formulae (D), (E), (F) have the following formulae:

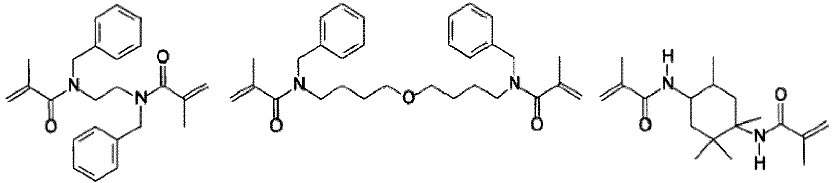

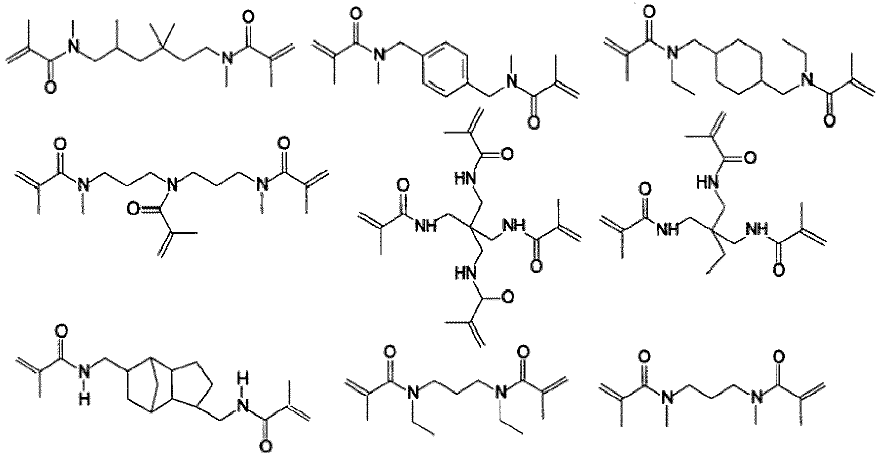

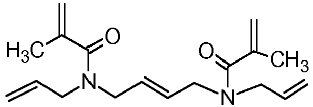

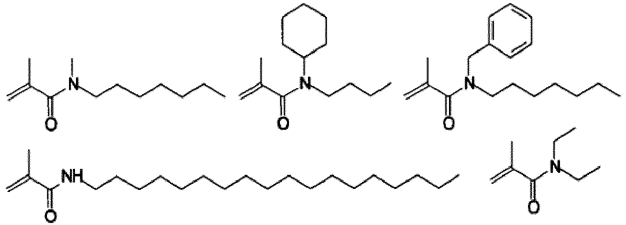

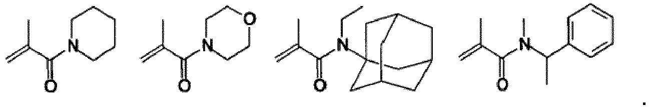

Preferred acrylamides according to formulae (D), (E), (F) have the following formulae:

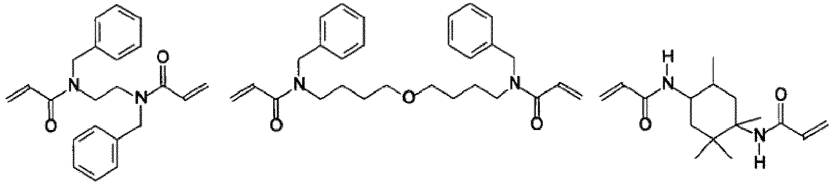

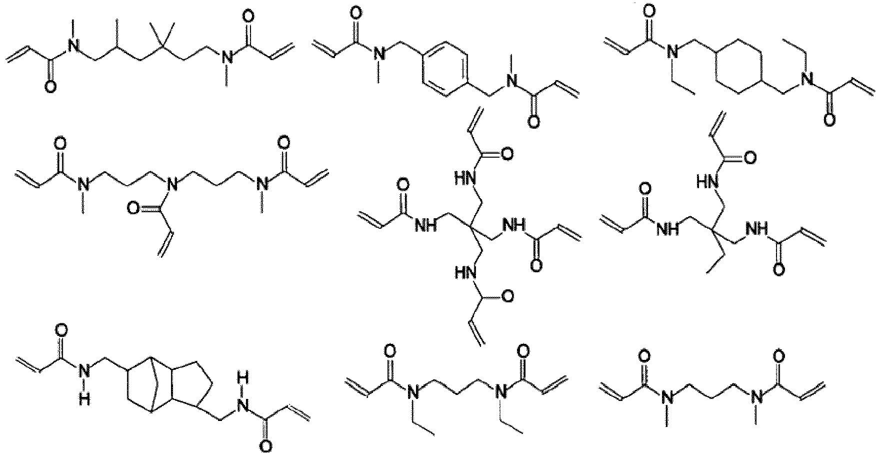

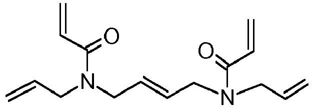

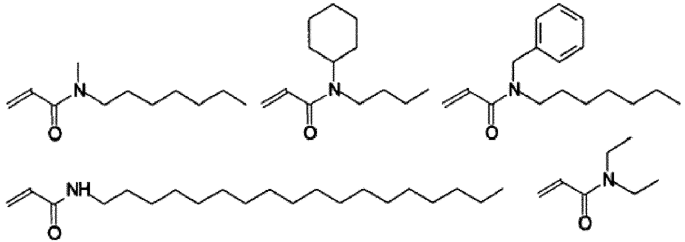

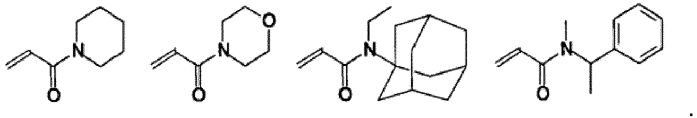

Most preferred are the bis-(meth)acrylamides:
N,N'-diallyl-1,4- bisacrylamido-(2E)-but-2-en (BAABE) having the structural formula 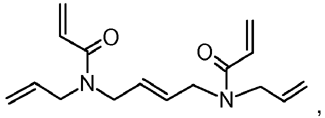
and N,N'-diethyl-1,3-bisacrylamido-propan (BADEP) having the structural formula

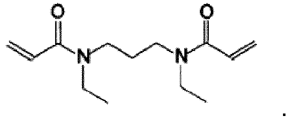

A polymerizable monomer (a) having a (meth)acryloyl group or a (meth)acrylamide group may also be selected from phosphoric acid ester group containing polymerizable monomers having at least one radically polymerizable double bond. Preferably, such phosphoric acid ester group containing polymerizable monomers have the following formula (G): 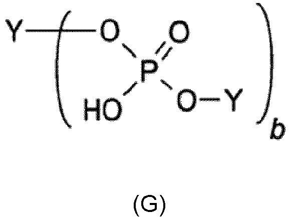 wherein
the moieties Y independent from each other represent a hydrogen atom or a moiety of the following formulae (Y*), (Y**) or (Y***): 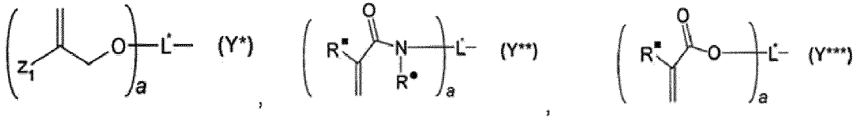 wherein
Z₁ is COOR^{α}, COSR^{β}, CON(R^{α})₂, CONR^{α}R^{β}, or CONHR^{α}, wherein R^{α} and R^{β} independently represent a hydrogen atom, a C₁₋₁₈ alkyl group optionally substituted by a C₃₋₈ cycloalkyl group, an optionally substituted C₃₋₈ cycloalkyl group, an optionally substituted C₄₋₁₈ aryl or heteroaryl group, an optionally substituted C₅₋₁₈ alkylaryl or alkylheteroaryl group, or an optionally substituted C₇₋₃₀ aralkyl group, whereby one R^{α} and one R^{β} residue may form together with the adjacent nitrogen atom to which they are bound a 5- to 7-membered heterocyclic ring which may contain further nitrogen atoms or an oxygen atoms, and whereby the optionally substituted groups may be substituted by 1 to 5 C₁₋₅ alkyl group(s);
R▪ and R• independently represent a hydrogen atom, an optionally substituted C₁₋₁₈ alkyl group, an optionally substituted C₃₋₁₈ cycloalkyl group, an optionally substituted C₅₋₁₈ aryl or heteroaryl group, an optionally substituted C₅₋₁₈ alkylaryl or alkylheteroaryl group, an optionally substituted C₇₋₃₀ aralkyl group, whereby the optionally substituted groups may be substituted by 1 to 5 C₁₋₅ alkyl group(s);
L* represents an (*a*+*b*)-valent organic residue (whereby b is 1 when Y in formula (G) is within the round brackets) containing 2 to 45 carbon atoms and optionally heteroatoms such as oxygen, nitrogen and sulfur atoms, the carbon atoms including *a* + *b* carbon atoms selected from primary and secondary aliphatic carbon atoms, secondary alicyclic carbon atoms, and aromatic carbon atoms, each of the a+b carbon atoms linking a phosphate or a moiety of any one of formula (Y*), (Y**) and (Y***); a is an integer of from 1 to 10, preferably 1 to 5; *b* is an integer of from 1 to 10, preferably 1 to 5; provided that at least one Y is not hydrogen. The preparation of such compounds wherein Y = Y* is known from EP 1 548 021 A1.

Furthermore, a polymerizable monomer (a) having a (meth)acryloyl group or a (meth)acrylamide group may also be selected from phosphonic acid group containing polymerizable acidic compounds of the following formula (H): 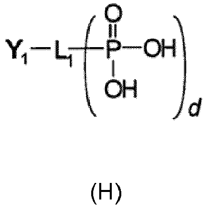 wherein
the moiety Y₁ represents a moiety of the following formulae (Y₁**) or (Y₁***): 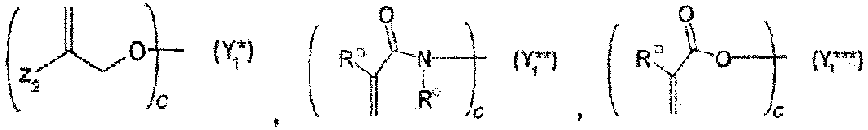
Z₂ independently has the same meaning as defined for Z₁;
R^{□} and R° independently have the same meaning as defined for R▪ and R•;
L₁ represents a (*c* + *d*) valent organic residue containing 2 to 45 carbon atoms and optionally heteroatoms such as oxygen, nitrogen and sulfur, the carbon atoms including c + *d* carbon atoms selected from primary and secondary aliphatic carbon atoms, secondary alicyclic carbon atoms, and aromatic carbon atoms, each of the c+d carbon atoms linking a phosphonate or a moiety of any one of formula (Y₁*), (Y₁**) and (Y₁***); and
c and *d* independently represent integers of from 1 to 10.

From compound of formula (G'), the following formulae are particularly preferred: 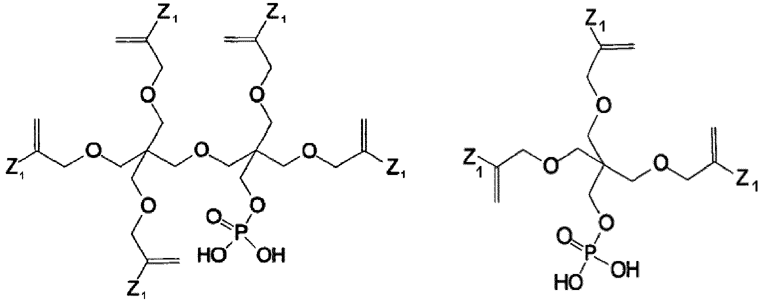 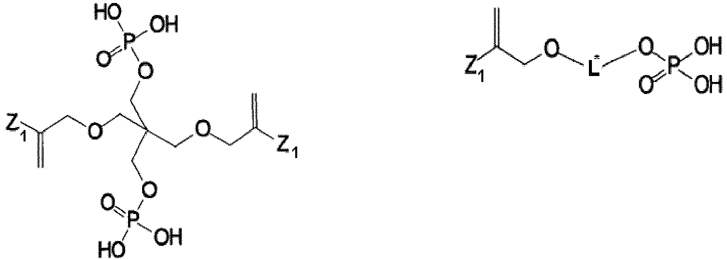 wherein Z₁ is defined as above, and L* is an optionally substituted alkylene group. More preferably, Z₁ is methyl, and L* is a C₄ to C₁₆ alkylene group. Even more preferably, L* is a C₈ to C₁₂ alkylene group.

Furthermore, a polymerizable monomer (a) having one or more radically polymerizable carbon-carbon double bond(s) may be selected from the hydrolysis stable polyfunctional polymerizable monomers disclosed in EP 2 705 827 and EP 2 727 576.

Particularly preferred polymerizable monomer(s) (a) are selected from the compounds of formulae (D), (E), (F), (G) and (H), more preferably from the compounds of formulae (D), (E), (F), and most preferably from compounds of formula (E).

Polymerizable monomer(s) (a) in the form of polymers are preferably selected from polymerizable polyacidic polymers.

The term "polymerizable" as used with the term "polymerizable polyacidic polymer" means a polymer capable of combining by covalent bonding in an addition polymerization. The "polymerizable polyacidic polymer" may be combined with a crosslinker as well as e.g. with a monomer having polymerizable (carbon-carbon) double bond, to form graft polymers and/or crosslinked polymers when curing the dental composition.

The term "polyacidic" as used with the term "polymerizable polyacidic polymer" means that the polymer has a plurality of acidic groups, preferably carboxylic acid groups, which may participate in a cement reaction with a reactive glass. The carboxylic acid groups are preferably present in the backbone and derived from acrylic acid, methacrylic acid and/or itaconic acid. Additional acidity may be introduced by carboxylic acid groups in the group of formula (V) and carboxylic group(s) in the optional repeating unit of formula (VI).

A particularly preferred polymerizable polyacidic polymer has repeating units of the following formula (IV): 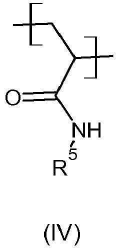 wherein R⁵ represents a group of the following formula (V):

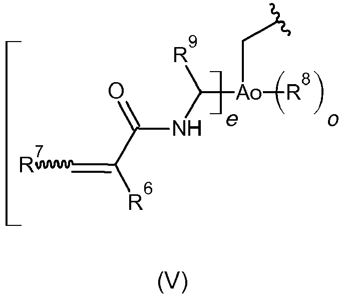

In formulae (V) and (IX), the jagged bond indicates that R⁷ may be in *cis* or *trans* configuration relative to the carbonyl group. Furthermore, in formula (V), the bond to the jagged line indicates the attachment of R⁵ to the nitrogen of the amide moiety of the repeating unit of formula (IV). In formula (VIII), the bond to the jagged line indicates the attachment of R¹¹ to the nitrogen of the amide moiety of the repeating unit of formula (VII).

The polymerizable polyacidic polymer having repeating units of formula (IV) is watersoluble and is reactive with a particulate glass in a cement reaction, whereby the polymerizable polyacidic polymer has a polymer backbone and hydrolysis-stable pendant groups R⁵ having one or more radically polymerizable carbon-carbon double bonds.

The polymerizable pendant groups R⁵ of the polymerizable polyacidic polymer having repeating units of formula (IV) may react with a monomer having a radically polymerizable double bond, whereby a graft polymer is formed. The grafted side-chains may contain additional carboxylic acid groups which can take part in a cement reaction, thereby further increasing the strength of the cured composition.

In formula (V), Ao is an aromatic group which may be further substituted. The aromatic group is not specifically limited and may be any organic aromatic group, i.e. a cyclic moiety which number of π-electrons equals 4*f*+2, where *f* is zero or any positive integer. Preferably, Ao is derived from an arene or heteroarene. An arene is a monoyclic or polycyclic aromatic hydrocarbon. A heteroarene is a heterocyclic compound formally derived from arenes by replacement of one or more methine (-C=) and/or vinylene (-CH=CH-) groups by trivalent or divalent heteroatoms, respectively, in such a way as to maintain the continuous π-electron system characteristic of aromatic systems and a number of out-of-plane π-electrons corresponding to the Hückel rule (4 *f* + 2).

In case o+e is 2, Ao is preferably a C₆₋₁₄ arenetriyl or C₃₋₁₄ heteroarenetriyl group which may be further substituted by one or more substituents. In case o + e is 3, Ao is preferably a C₆₋₁₄ arenetetrayl or C₃₋₁₄ heteroarenetetrayl group which may be further substituted by one or more additional substituents. In case o + e is 4, Ao is preferably a C₆₋₁₄ arenepentayl or C₃₋₁₄ heteroarenepentayl group which may be further substituted by one or more additional substituents. In case o + e is 5, then Ao is preferably a C₆₋₁₄ arenehexayl or C₃₋₁₄ heteroarenehexayl group which may be further substituted by one or more additional substituent.

The additional substituents are selected from the group consisting of a straight chain or branched C₁ to C₁₀ alkyl group, a straight chain or branched C₁ to C₁₀ alkenyl group, - COOM, -PO₃M, -O-PO₃M₂ and -SO₃M, wherein M represents a hydrogen atom or a metal atom. More preferably, Ao is a C₆₋₁₀ arenetriyl or C₃₋₉ heteroarenetriyl group which may be substituted by one or more additional substituents selected from a straight chain or branched C₁ to C₄ alkyl group and a straight chain or branched C₁ to C₄ alkenyl group.

Even more preferably, Ao is selected from a benzenetriyl group, a naphtalenetriyl group, a toluenetriyl group, a xylenetriyl group and a styrenetriyl group, and the heteroaryl group is a pyridinetriyl group. Yet even more preferably, Ao is a benzenetriyl group. Most preferably, Ao is a benzenetriyl group wherein a hydroxyl group is present in formula (V) in para-position to the methylene group linking R⁵.

In formula (V), R⁶ and R⁷, which may be the same or different, independently represent a hydrogen atom, or a C₁₋₆ alkyl group which may be substituted with a carboxylic acid group. Preferably, R⁶ and R⁷, which may be the same or different, independently represent a hydrogen atom or a C₁₋₃ alkyl group. More preferably, R⁶ represents a hydrogen atom or a methyl group, and R⁷ represents a hydrogen atom. Most preferably, both R⁶ and R⁷ represent a hydrogen atom.

In formula (V), R⁸ represents an electron donating group which activates the aryl group. Accordingly, each R⁸ is directly bonded to a ring atom of the Ao group. R⁸ may be a halogen atom or a group selected from -OH, -OR^{d}, -NR^{e}H, -NR^{e}R^{f}, -SH, and -SR9, wherein R^{d}, R^{e}, R^{f}, R^{g}, and R^{g}, represent a C₁₋₆ alkyl group. Preferably, R⁸ is a hydroxyl group. The halogen atom may be a fluorine atom, a chlorine atom, a bromine atom or an iodine atom. When o is 2, the R⁸ cannot be both OH.

In formula (V), one or more R⁹ may be present depending on the value of e. The R⁹ may be the same or different when more than one R⁹ is present. R⁹ represents a hydrogen atom, a carboxylic acid group, a COOR^{a} group, a CONHR^{b} group, or a CONR^{c}₂ group. R^{a}, R^{b}, and R^{c} represent a C₁₋₆ alkyl group. According to a preferred embodiment, R⁹ represents a hydrogen atom.

In formula (V), o is an integer of 1 or 2. Preferably, o is 1. In formula (V), e is an integer of 1 to 4. Preferably, e is an integer of 1 or 2. In formula (V), o+e is preferably 5 or less, more preferably 4 or less, in particular 3.

It is preferred that in formula (V), Ao is a phenyl group. Specifically, R⁵ preferably represents a group of the following formula (V'): 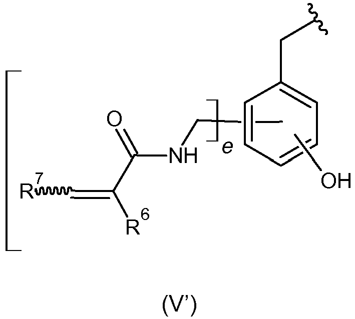 wherein R⁶, R⁷ and e are as defined as above.

It is particularly preferred that R⁵ is a group of the following formula (V"ₐ) or (V"_{b}):

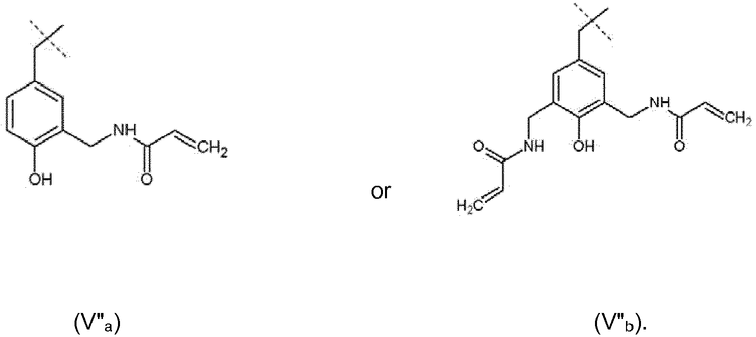

Furthermore, it is preferred that the polymerizable polyacidic polymer having repeating units of formula (IV) further comprises acidic repeating units of the following formula (VI):

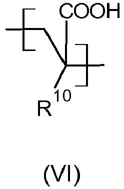

In formula (VI), R¹⁰ represents a hydrogen atom, or a C₁₋₆ alkyl group which may be substituted with a carboxylic acid group. Preferably, R¹⁰ represents a hydrogen atom, or a C₁₋₃ alkyl group which may be substituted with a carboxylic acid group, more preferably R¹⁰ represents a hydrogen atom or a methyl group. Most preferably, R¹⁰ represents a hydrogen atom.

In the polymerizable polyacidic polymer having repeating units of formula (IV), the molar ratio of repeating units of formula (VI) and repeating units of formula (IV) ([ formula (VI) ]/[ formula (IV)]) is preferably in the range of 1000:1 to 1:1, more preferably 100:1 to 5:1, most preferably 50:1 to 10:1.

The polymerizable polyacidic polymer having repeating units of formula (IV) preferably has a molecular weight M_{w} in the range of 10,000 to 250,000, more preferably 20,000 to 150,000, most preferably 30,000 to 100,000.

The polymerizable polyacidic polymer having repeating units of formula (IV) is hydrolysis stable, which means that it does not contain groups hydrolysing at pH 2.5 within one month when stored at a temperature of 50 °C.

According to a particularly preferred embodiment, the polymerizable polyacidic polymer has repeating units of the following formula (IV): 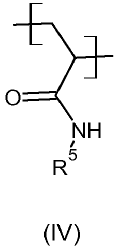 wherein R⁵ represents a group of the following formula (V'): 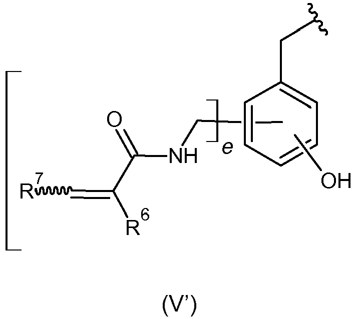 wherein
R⁶ and R⁷, which may be the same or different, independently represent a hydrogen atom, or a C₁₋₄ alkyl group; preferably R⁶ is a hydrogen atom or a methyl group and R⁷ is a hydrogen atom, and
e is an integer of 1 to 3, preferably n is an integer of 1 or 2,
which polymerizable polyacidic polymer further comprises acidic repeating units of the following formula (VI): 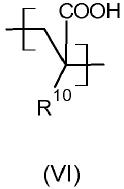
wherein
   - R¹⁰: represents a hydrogen atom, or a C₁₋₄ alkyl group, preferably R¹⁰ represents a hydrogen atom or a methyl group
wherein the molar ratio of repeating units of formula (VI) and repeating units of formula (IV) ([formula (VI) ]/[ formula (IV)]) is in the range of 100:1 to 5:1, preferably 50:1 to 10:1, and the molecular weight M_{w} is in the range of 20,000 to 150,000, preferably 30,000 to 100,000.

The process for preparing a polymerizable polyacidic polymer having repeating units of formula (IV) comprises reacting a polyacidic polymer having repeating units of the following formula (VII): 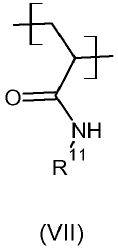 wherein R¹¹ represents a group of the following formula (VIII): 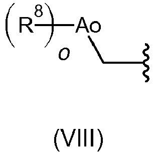 wherein
- Ao: is an aromatic group which may be further substituted;
- R⁸: represents a halogen atom or a group selected from -OH, -OR^{d}, -NR^{e}H, -NR^{e}R^{f}, -SH, and -SR^{g}, wherein R^{d}, R^{e}, R^{f}, R^{g}, and R^{g}, represent a C₁₋₆ alkyl group; and
- o: is an integer of 1 or 2, provided that when o is 2, the R⁸ cannot be both OH,
with a compound of the following formula (IX) 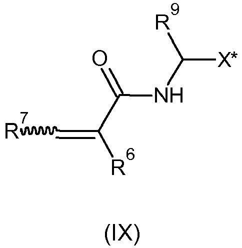 wherein X* is a leaving group, and
R⁹, R⁶ and R⁷, which may be the same or different, independently represent a hydrogen atom, or a C₁₋₆ alkyl group which may be substituted with a carboxylic acid group.

In compound of formula (IX), leaving group X* is preferably a leaving group susceptible to C-C bond-formation by means of electrophilic aromatic substitution. More preferably, leaving group X* is selected from the group consisting of a fluorine atom, a chlorine atom, a bromine atom or a hydroxyl group. Most preferably, leaving group X* is a hydroxyl group.

The reaction conditions for polymer analogous reaction of the polyacidic polymer having repeating units of the formula (VII) with a compound of formula (IX) are not particularly limited.

Preferably, the reaction is carried out in the presence of a solvent. More preferably, the solvent is water.

The reaction temperature for reacting the polyacidic polymer having repeating units of formula (VII) with a compound of formula (IX) is not particularly limited. Preferably, the reaction is carried out at a temperature of between 20 to 90°C. Most preferably, the reaction temperature is in the range of from 40 to 80°C.

The reaction time for reacting the polyacidic polymer having repeating units of formula (VII) with a compound of formula (IX) is not particularly limited. Preferably, the reaction time is in the range of from 1 to 72 hours, most preferably 12 to 50 hours.

The molar ratio of polyacidic polymer having repeating units of formula (VII) to compound of formula (IX) is not particularly limited. Preferably, the molar ratio of polyacidic polymer having repeating units of formula (VII) to compound of formula (IX) is 1:5 to 1:1000, more preferably 1:100 to 1: 800, most preferably 1:300 to 1:700.

Reacting of the polyacidic polymer having repeating units of formula (VII) with a compound of formula (IX) may be carried out in the presence of a catalyst, preferably a catalyst in the form of an organic or inorganic acid. More preferably, the catalyst is selected from the group consisting of hydrochloric acid, hydrobromic acid, hydrofluoric acid, phosphoric acid, sulphuric acid, sulfamic acid, oxalic acid and p-toluenesulfonic acid. Most preferably, the catalyst is hydrochloric acid or oxalic acid. The amount of catalyst may be selected from 0.01 to 100 mol%, preferably from 10 to 90 mol%, more preferably from 30 to 80 mol% based on the molar amount of the polyacidic polymer having repeating units of formula (VII) and compound of formula (IX).

The number e of groups of formula (V) in R⁵ of the reaction product in the form of the polymerizable polyacidic polymer having repeating units of formula (IV) may be set by suitably selecting the reaction conditions for reacting the polyacidic polymer having repeating units of formula (VII) with the compound of formula (IX). For example, for setting e = 1, oxalic acid may be applied as the catalyst, and the reaction temperature is preferably within a range of 60 to 80 °C. For setting e = 2, hydrochloric acid may be applied as the catalyst, and the reaction temperature is preferably within a range of 35 to 55 °C.

The reaction product obtained from reacting the polyacidic polymer having repeating units of formula (VII) with a compound of formula (IX) may be purified according to conventional methods. Preferably, the reaction product in the form of the polymerizable polyacidic polymer having repeating units of formula (IV) is separated from the reaction mixture and purified by dialysis against water, more preferably the dialysis is carried out with a size exclusion of molecules having a molecular weight of up to 2000 g/mol. Owing to the purification by means of dialysis, or well-known polymer-chemically purification methods such as precipitation, liquid-liquid extraction. The polymerizable polyacidic polymer having repeating units of formula (IV) is obtained in both high yields and purity.

According to a particularly preferred embodiment, the process for preparing a polymerizable polyacidic polymer having repeating units of formula (IV) comprises reacting a polyacidic polymer having repeating units of the following formula (VII): 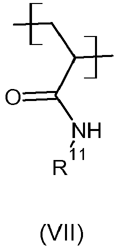 wherein R¹¹ represents a group of the following formula (Villa): 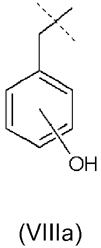 with a compound of the following formula (Vla) 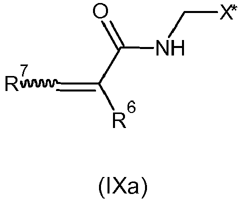
wherein X* is a hydroxyl group, R⁶ is a hydrogen atom or a methyl group, preferably a hydrogen atom, and R⁷ is a hydrogen atom,
in water as the solvent and in the presence of a catalyst selected from the group consisting of hydrochloric acid, hydrobromic acid, hydrofluoric acid, phosphoric acid, sulphuric acid, sulfamic acid, oxalic acid and p-toluenesulfonic acid, preferably the catalyst is hydrochloric acid or oxalic acid,
wherein the amount of catalyst may be selected from 10 to 90 mol%, preferably from 30 to 80 mol% based on the molar amount of the polyacidic polymer having repeating units of formula (VII) and compound of formula (IXa),
wherein the reaction temperature is in the range of from 40 to 80°C, and
the molar ratio of polyacidic polymer having repeating units of formula (VII) to compound of formula (IXa) is 1:100 to 1: 800, preferably 1:300 to 1:700.

A starting material in the form of the polyacidic polymer having repeating units of formula (VII) may be provided by polymerizing a monomer represented by the following formula (VII): 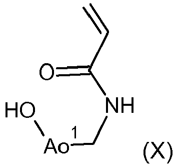 wherein
Ao¹ is an aromatic group as Ao defined above for formula (V). Alternatively, the substitution pattern of the aromatic group may be adapted to the desired copolymer.

Preferably, the starting material in the form of the polyacidic polymer having repeating units of formula (VII) is an acrylic acid derivative copolymer having repeating units of formulae (VII) and (VI) which may be obtained by copolymerizing a monomer represented by the following formula (X): 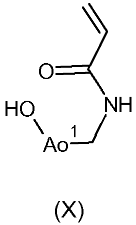 wherein
Ao¹ is an aromatic group as defined above,
with a monomer represented by the following formula (XI) 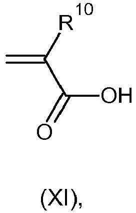
wherein R¹⁰ is defined as above for formula (VI).

The carboxylic acid group(s) optionally comprised in the monomer represented by formula (X) and/or comprised in the monomer represented by formula (XI) may optionally be protected.

The protecting group of an optionally protected carboxylic acid group is not particularly limited as long as it is a carboxyl-protecting group known to those of ordinary skill in the art of organic chemistry (cf. P.G.M. Wuts and T.W. Greene, Greene's Protective Groups in Organic Synthesis, 4th Edition, John Wiley and Sons Inc., 2007). Preferably, the carboxyl-protecting group is selected from a trialkylsilyl group, an alkyl group and an arylalkyl group. More preferably, the carboxyl-protecting group is selected from an alkyl group or an arylalkyl group. Most preferably, the carboxyl-protecting group is selected from a *tert*-butyl group and a benzyl group. In one preferred embodiment, the carboxyl-protecting group is a *tert*-butyl group.

The optionally protected carboxylic acid group(s) can be deprotected prior to polymerization or copolymerization of the monomer represented by formula (X), concomitant thereto or subsequently thereto.

The conditions for deprotection of the optionally protected carboxylic acid group(s) are selected according to the protecting group used. Preferably, the protected carboxylic acid group(s) is/are deprotected by hydrogenolysis or treatment with acid or base.

If the deprotection of the optionally protected carboxylic acid group(s) is carried out concomitantly with polymerization or copolymerization of the monomer represented by formula (X), it will be understood by a person skilled in the art that the deprotection conditions and the conditions for the polymerization or copolymerization have to be selected so that both reactions can proceed efficiently.

The reaction conditions for polymerizing or copolymerizing the monomer represented by formula (X) are not particularly limited. Accordingly, it is possible to carry out the reaction in the presence or absence of a solvent. Preferably, the reaction is carried out in the presence of a solvent. A suitable solvent may be selected from the group of water, dimethyl formamide (DMF), tetrahydrofurane (THF), and dioxane. Preferably, the solvent is dioxane.

The reaction temperature for polymerizing or copolymerizing the monomer represented by formula (X) is not particularly limited. Preferably, the reaction is carried out at a temperature of between -10°C to the boiling point of the solvent. More preferably, the reaction temperature is in the range of from 0 to 110°C, even more preferably 40 to 100 °C, most preferably 60 to 90°C.

The reaction time for polymerizing or copolymerizing the monomer represented by formula (X) is not particularly limited. Preferably, the reaction time is in the range of from 10 minutes to 48 hours, more preferably 1 hour to 36 hours, even more preferably 2 to 24 hours, most preferably 3 to 12 hours.

The reaction for polymerizing or copolymerizing the monomer represented by formula (X) is preferably carried out in the presence of a polymerization initiator. Preferably, the polymerization initiator is selected from azobisisobutyronitrile (AIBN), 2,2-azobis(2-amidinopropane)dihydrochloride, 2,2'-azobis(2-methylbutyronitrile), 2,2'-azobis(N,N'-dimethyleneisobutyramidine) dihydrochloride, and 4,4'-azobis(4-cyano pentanoic acid), most preferably, the polymerization initiator is AIBN. The amount of the polymerization initiator is not particularly limited. Suitably, the amount is in the range of from 0.001 to 5 mol % based on the total amount of the monomers.

The reaction for copolymerizing the monomer represented by formula (X) and the monomer represented by formula (XI) is preferably carried out by providing the monomer represented by the formula (X) and the monomer represented by the formula (XI) in the molar ratio ([ formula (X) ]/[ formula (XI)]) in the range of 1000:1 to 1:1, more preferably 100:1 to 5:1, most preferably 50:1 to 10:1.

In the acrylic acid derivative copolymer having repeating units of formulae (VII) and (VI), the molar ratio of repeating units of formula (VI) and repeating units of formula (VII) ([ formula (VI) ]/[ formula (VII)]) is preferably in the range of 1000:1 to 1:1, more preferably 100:1 to 5:1, most preferably 50:1 to 10:1.

The reaction product obtained from polymerizing or copolymerizing the monomer represented by formula (X) may be isolated by precipitation and filtration, or lyophilisation, preferably by precipitation and filtration. The reaction product may be purified according to conventional methods. It was surprisingly found that the reaction product can be obtained in both high yield and purity simply by dissolving and precipitating the reaction product, preferably twice. Hence, it can be dispensed with elaborate purification of the reaction product. For example, the crude reaction product may be dissolved in a suitable organic solvent, e.g. in dioxane, and precipitated by adding a suitable organic solvent, e.g. acetonitrile.

The acrylic acid derivative copolymer having repeating units of formulae (VII) and (VI) may be a statistical copolymer, a random copolymer, an alternating copolymer, a block copolymer or a combination thereof. Preferably, it is a statistical copolymer.

Preferably, in the acrylic acid derivative copolymer having repeating units of formulae (VII) and (VI), R¹¹ represents a group of the following formula (V'):

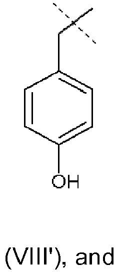

R¹⁰ represents a hydrogen atom.

The monomer represented by the formula (X) may be prepared by reacting a compound of the following formula (XII) 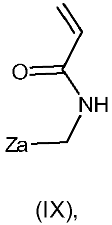 wherein Za is a leaving group,
with a compound of formula (XIII)

Ao²-OH (XIII),

wherein Ao² is an aromatic group as defined above for formula (V).

Preferably, leaving group Za of compound of formula (XII) is a leaving group susceptible to C-C bond-formation by means of electrophilic aromatic substitution. More preferably, leaving group Za is selected from the group consisting of a fluorine atom, a chlorine atom, a bromine atom or a hydroxyl group. Most preferably, leaving group Za is a hydroxyl group.

The reaction conditions for reacting the compound of formula (XII) with the compound of formula (XII) are not particularly limited.

The reaction may be carried out in the absence or presence of a solvent, preferably in the presence of a solvent. The solvent is preferably selected from the group consisting of acetone, THF, ethyl acetate, chloroform, 1,2-dichlorethane. Most preferably, the solvent is acetone.

The reaction temperature for reacting the compound of formula (XII) with the compound of formula (XIII) is not particularly limited. Preferably, the reaction is carried out at a temperature of between -10 to 70°C. More preferably, the reaction temperature is in the range of from 10 to 60°C, most preferably from 30 to 50 °C.

The reacting of the compound of formula (XII) with the compound of formula (XIII) may be carried out in the presence of a catalyst, preferably in the form of an organic or inorganic acid. More preferably, the catalyst is an inorganic Lewis acid, that is an inorganic electron acceptor. Even more preferably, the catalyst is selected from the group consisting of AlCl₃, BF₃, FeCl₃, FeCl₂, FeBr₃, FeBr₂, FeSO₄, Fe₂(SO₄)₃, ZnCl₂, ZnBr₂, ZnSO₄. Yet even more preferably, the catalyst is selected from the group consisting of AlCl₃, BF₃ and FeCl₃. Most preferably, the catalyst is AlCl₃. The amount of catalyst may be selected from 0.01 to 150 mol%, preferably from 30 to 130 mol%, more preferably from 60 to 120 mol%, most preferably from 90 to 110 mol% based on the molar amount of compound of formula (XII).

Furthermore, when reacting the compound of formula (XII) with the compound of formula (XIII), an antioxidant may be added which suppresses polymerisation and/or autoxidation of compound of formula (XII). Preferably, the antioxidant is selected from the group consisting of 3,5-die-tert-4-butylhydroxytoluene (BHT), 4-tert-butylcatechol, phenothioazine, tert.-butyl hydroquinone (TBHQ) and hydroxytoluene. Most preferably, the antioxidant is phenothioazine. The amount of antioxidant may be selected from 0.001 to 2% and preferably from 0.02 to 0.5% based on the total weight of compound of formula (XII).

The reacting of the compound of formula (XII) with the compound of formula (XIII) is not particularly limited. Preferably, the reaction time is in the range of from 10 minutes to 48 hours, more preferably 1 hour to 36 hours, most preferably 2 to 24 hours.

The product obtained by reacting the compound of formula (XII) with the compound of formula (XIII) may be isolated from the crude reaction mixture by extraction with an organic solvent, preferably chloroform or dichloromethane. The product may be purified according to conventional methods, preferably by silica-gel column chromatography.

### The initiator system (b) comprising (b-1) and (b-2)

### The sensitizer or oxidizing agent capable of oxidizing a reducing agent of a redox initiator system (b-1)

### The sensitizer (b-1)

The initiator system (b) may be a photoinitiator system which comprises a sensitizer (b-1).

Suitable sensitizers (b-1) are alpha diketones that have light absorption within a range of about 400 nm to about 520 nm (even more preferably, about 450 to about 500 nm). Examples include camphor quinone, benzil, furil, 3,3,6,6-tetramethylcyclo-hex-anedione, phenanthraquinone, 1-phenyl-1,2-propanedione and other 1-aryl-2-alkyl-1,2-ethanediones, and cyclic alpha diketones.

### The oxidizing agent capable of a redox initiator system (b-1)

The initiator system (b) may be a redox initiator system. Accordingly, the initiator system comprises an oxidizing agent capable of oxidizing a reducing agent of a redox initiator system (b-1). The oxidizing agent capable of oxidizing a reducing agent of a redox initiator system is preferably a peroxide or hydroperoxide.

Preferably, the peroxide or hydroperoxide is selected from cumyl hydroperoxide, tert-butyl peroxybenzoate, tert-butylperoxy (2-ethylhexyl)carbonate, tert-butylhydroperoxide, di(tert-butyl)peroxide, tert-butylperoxy-3,5,5-trimethyl-hexanoate and potassium peroxydisulfate. More preferably, the peroxide or hydroperoxide is selected from cumyl hydroperoxide, tert-butyl peroxybenzoate and potassium peroxydisulfate. Most preferably, the peroxide or hydroperoxide is potassium peroxydisulfate.

Optionally, the redox initiator system comprises one or more inorganic catalysts and/or organic polymerization accelerators.

Preferably, the catalyst is a metal salt, more preferably a transition metal salt. Even more preferably, the catalyst is a transition metal salt of V, Fe, Cu, Ti, Mn, Ni and Zn. Most preferably, the catalyst is a transition metal salt of Fe or Cu. Tetravalent and/or penta-valent vanadium compounds are preferred, including vanadium(IV) oxide, vanadyl(IV) acetylacetonate, vanadyl(IV) oxalate, vanadyl(IV) sulfate, oxobis(1-phenyl-1,3-butanedi-onato)vanadium(IV), bis(maltolato)oxovanadium(IV), vanadium(V) oxide, sodium metavanadate(V), and ammonium metavanadate(V). Examples of the copper compounds include copper acetylacetonate, copper(II) acetate, copper oleate, copper(II) chloride, and copper(II) bromide.

Preferably, the polymerization accelerator is selected from sulfinic acids, sulfinates, sulfites, hydrogen sulfites, aldehydes, thiourea compounds, barbituric acid derivatives, triazine compounds, halogen compounds, and thiol compounds

Examples of the sulfinic acids and sulfinates that may be used as the polymerization accelerator include p-toluenesulfinic acid, sodium p-toluenesulfinate, potassium p-toluenesulfinate, calcium p-toluenesulfinate, benzenesulfinic acid, sodium benzenesulfinate, potassium benzenesulfinate, calcium benzenesulfinate, 2,4,6-trimethylbenzenesulfinic acid, sodium 2,4,6-trimethylbenzenesulfinate, potassium 2,4,6-trimethylbenzenesulfinate, calcium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, calcium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, and calcium 2,4,6-triisopropylbenzenesulfinate. Among these, sodium p-toluenesulfinate, sodium benzenesulfinate, and sodium 2,4,6-triisopropylbenzenesulfinate are preferred. Examples of the sulfites and hydrogen sulfites that may be used as the polymerization accelerator include sodium sulfite, potassium sulfite, calcium sulfite, ammonium sulfite, sodium hydrogen sulfite, and potassium hydrogen sulfite.

Examples of the aldehydes that may be used as the polymerization accelerator include terephthalaldehydes; and benzaldehyde derivatives such as dimethylaminobenzaldehyde, p-methyloxybenzaldehyde, p-ethyloxybenzaldehyde, and p-n-octyloxybenzaldehyde.

Examples of the thiourea compounds that may be used as the polymerization accelerator include 1-(2-pyridyl)-2-thiourea, thiourea, methylthiourea, ethylthiourea, N,N'-dimethyl-thiourea, N,N'-diethylthiourea, N,N'-di-n-propylthiourea, N,N'-dicyclohexylthiourea, trimethylthiourea, triethylthiourea, tri-n-propylthiourea, tricyclohexylthiourea, tetramethyl-thiourea, tetraethylthiourea, tetra-n-propylthiourea, tetracyclohexylthiourea, 3,3-dimethylethylenethiourea, and 4,4-dimethyl-2-imidazolinethione.

The content of the polymerization accelerator is preferably 0.01 to 5 wt%, and more preferably 0.05 to 3 wt% with respect to the total dental polymerizable composition of the present invention.

### The coinitiator (b-2)

A coinitiator (b-2) according to the present invention is a coinitiator of the following formula (I):

Q-X (I)

wherein
- Q: is a cyano group or a group A-Q'-;
- wherein A: is a hydrogen atom, a further group X, or a polymerizable group having a carbon-carbon double bond copolymerizable with the polymerizable monomer;
- Q': is an aliphatic hydrocarbon group or a polyoxyalkylene group, which have two to twelve carbon atoms and which may have up to two carbon-carbon double bonds and/or polymerizable groups having a carbon-carbon double bond, which are co-polymerizable with the polymerizable monomer, and which may be substituted with a group, preferably selected from a hydroxy group;
- X: which may be the same or different when more than one X is present, independently represents a group of the following formula (IIa) when Q is a cyano group or a group of the following formula (lib) when Q is A-Q'-:

-Ar(R¹)ₙ-NR²R³ (IIa)

-X¹C(O)X²Ar(R¹)ₙ-NR²R³ (IIb)

wherein
X¹ and X² which may be the same or different, independently represent a single bond, -NR⁴- , -O-, or -S-, wherein R⁴ is a hydrogen atom, a C₁₋₆ alkylene group, a C₃₋₆ cycloalkylene group or an allyl group;
Ar is an (n+2)-valent aromatic hydrocarbon group;
R¹ which may be the same or different if more than one R¹ is present, independently represents a monovalent substituent having a polymerizable carbon-carbon double bond copolymerizable with the polymerizable monomer, a cyano group, a C₁₋₆ alkyl group, a C₁₋₆ alkoxy group, a halogen atom and nitro group;
n is 0 or an integer of from 1 to 4;
R² and R³, which may be the same or different, independently represent a saturated aliphatic hydrocarbon group having one to eight carbon atoms, an allyl group, or R² and R³ form together with the nitrogen atom to which they are bonded a C₃₋₆ saturated carbocyclic group which may contain a heteroatom selected from an oxygen atom and a sulfur atom, or R² and R³ form together with the nitrogen atom to which they are bonded a cyclic group further containing in the ring a group of the following formula (III)

   >NAr(R¹)ₙX²C(O)X¹Q, (III)
wherein Ar, R¹, n, X²; X¹ and Q, independently are as defined above;
provided that the compound of formula (I) contains at least one polymerizable carbon-carbon double bond copolymerizable with the polymerizable monomer and/or at least two groups selected from X and a group of formula (III).

In formula (I), Q is a cyano group or a group A-Q'-. When Q is a cyano group, then X is a group of the formula (Ila). When Q is a group A-Q', then X is a group of the formula (IIb).

In a preferred embodiment thereof in which Q is a cyano group, the coinitiator (b-2) of formula (I) according to the present invention is a compound of the following formula (Q1):

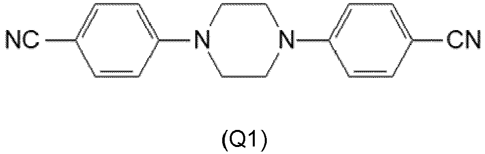

The moiety A may be a hydrogen atom, a further group X, or a polymerizable group having a carbon-carbon double bond copolymerizable with the polymerizable monomer. Preferably, the moiety A is a further group X or a polymerizable group having a carbon-carbon double bond copolymerizable with the polymerizable monomer. More preferably, A is a polymerizable group having a carbon-carbon double bond copolymerizable with the polymerizable monomer.

In a specific embodiment, wherein A is a polymerizable group having a carbon-carbon double bond copolymerizable with the polymerizable monomer, the polymerizable group having a carbon-carbon double bond may be a vinyl group, an allyl group, a (meth)acryloyl group, a (meth)acrylamide group or an allyl (meth)acrylamide group, more preferably a vinyl group, a (meth)acryloyl group or an allyl (meth)acrylamide group.

Q' is a divalent aliphatic hydrocarbon group or a polyoxyalkylene group, which groups may have two to twelve carbon atoms and which may have up to two carbon-carbon double bonds and/or polymerizable groups having a carbon-carbon double bond, which are copolymerizable with the polymerizable monomer, and which may be substituted with a group selected from a hydroxy group, preferably Q' is an aliphatic hydrocarbon group which may have two to twelve carbon atoms and which may have up to two carbon-carbon double bonds and/or polymerizable groups having a carbon-carbon double bond, which are copolymerizable with the polymerizable monomer, and which may be substituted with a group selected from a hydroxy group.

In a preferred embodiment, Q' is a polyoxyalkylene group, which may have two to twelve carbon atoms and which may have up to two carbon-carbon double bonds and/or polymerizable groups having a carbon-carbon double bond, which are copolymerizable with the polymerizable monomer, and which may be substituted with a group selected from a hydroxy group. Preferably, Q' is a polyoxyalkylene group which may have four to twelve carbon atoms, more preferably Q' is a polyoxyalkylene group which may have six to ten carbon atoms, and even more preferably Q' is a polyoxyalkylene group having ten carbon atoms.

Preferably, Q' is a polyoxyalkylene group which may have up to two carbon-carbon double bonds and/or polymerizable groups having a carbon-carbon double bond, which are copolymerizable with the polymerizable monomer, more preferably Q' is a polyoxyalkylene group having no carbon-carbon double bonds or polymerizable groups having a carbon-carbon double bond, which are copolymerizable with the polymerizable monomer, and even more preferably Q' is a polyoxyalkylene group having no carbon-carbon double bonds. Preferably, Q' is a polyoxyalkylene group which may be substituted with up to four groups selected from a hydroxy group, more preferably Q' is a polyoxyalkylene group which is substituted with up to two groups selected from a hydroxy group.

In another preferred embodiment, Q' is a polyoxyalkylene having up to two polymerizable groups having a carbon-carbon double bond, which are copolymerizable with the polymerizable monomer, wherein the polymerizable group is a vinyl group, an allyl group, a (meth)acryloyl group, a (meth)acrylamide group or an allyl (meth)acrylamide group, more preferably a vinyl group, a (meth)acryloyl group or an allyl (meth)acrylamide group.

In a particularly preferred embodiment, Q' is a polyoxyalkylene group having the following formula:

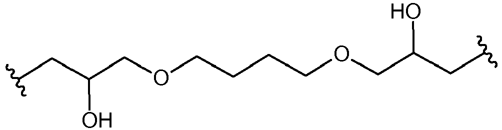

In a particularly preferred embodiment, Q' is an aliphatic hydrocarbon group which may have two to twelve carbon atoms and which may have up to two carbon-carbon double bonds and/or polymerizable groups having a carbon-carbon double bond, which are copolymerizable with the polymerizable monomer, and which may be substituted with a group selected from a hydroxy group. Preferably, Q' is an aliphatic hydrocarbon group which may have two to twelve carbon atoms, more preferably two to eight carbon atoms, even more preferably two to four carbon atoms, most preferably two carbon atoms.

Preferably, Q' is an aliphatic hydrocarbon group which may have up to two carbon-carbon double bonds and/or polymerizable groups having a carbon-carbon double bond, which are copolymerizable with the polymerizable monomer, more preferably Q' is an aliphatic hydrocarbon group having no carbon-carbon double bonds and up to two polymerizable groups having a carbon-carbon double bond, which are copolymerizable with the polymerizable monomer, and even more preferably Q' is an aliphatic hydrocarbon group having no carbon-carbon double bonds and no polymerizable groups having a carbon-carbon double bond. Preferably, Q' is an aliphatic hydrocarbon group which may be substituted with up to four groups selected from a hydroxy group, more preferably Q' is an aliphatic hydrocarbon group which may be substituted with up to two groups selected from a hydroxy group, even more preferably Q' an aliphatic hydrocarbon group which is not substituted with a group selected from a hydroxy group.

In another preferred embodiment, Q' is an aliphatic hydrocarbon group having up to two polymerizable groups having a carbon-carbon double bond, which are copolymerizable with the polymerizable monomer, wherein the polymerizable group is a vinyl group, an allyl group, a (meth)acryloyl group, a (meth)acrylamide group or an allyl (meth)acrylamide group, more preferably a vinyl group, a (meth)acryloyl group or an allyl (meth)acrylamide group, even more preferably a (meth)acryloyl group.

In a particularly preferred embodiment, Q' is an aliphatic hydrocarbon group having one of the following formulae:

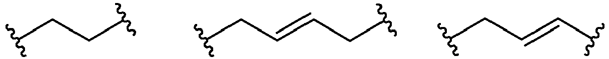

In formula (I), X which may be the same or different when more than one X is present, independently represents a group of the following formula (Ila) or (lib):

-Ar(R¹)ₙ-NR²R³ (IIa)

-X¹C(O)X²Ar(R¹)ₙ-NR²R³ (IIb)

wherein
X¹ and X², which may be the same or different, independently represent -NR⁴- , -O-, or - S-, wherein R⁴ is a hydrogen atom, a C₁₋₆ alkylene group, a C₃₋₆ cycloalkylene group or an allyl group;
Ar is an (n+2)-valent aromatic hydrocarbon group;
R¹ which may be the same or different if more than one R¹ is present, independently represents a monovalent substituent having a polymerizable carbon-carbon double bond copolymerizable with the polymerizable monomer, a cyano group, a C₁₋₆ alkyl group, a C₁₋₆ alkoxy group, a halogen atom and nitro group;
n is 0 or an integer of from 1 to 4;
R² and R³, which may be the same or different, independently represent a saturated aliphatic hydrocarbon group having one to eight carbon atoms, an allyl group, or R² and R³ form together with the nitrogen atom to which they are bonded a C₃₋₆ saturated carbocyclic group which may contain a heteroatom selected from an oxygen atom and a sulfur atom, or R² and R³ form together with the nitrogen atom to which they are bonded a cyclic group further containing in the ring a group of the following formula (III)

   >NAr(R¹)ₙX²C(O)X¹Q, (III)
wherein Ar, R¹, n, X², X¹ and Q, independently are as defined above;

Preferably, in formula (IIa) or (lib), Ar may be a o-, p- or m-phenylene group, more preferably a o- or a p-phenylene group, and even more preferable a p-phenylene group.

In formula (Ila) or (lib), R¹ may be the same or different when more than one R¹ is present. R¹ independently represents a monovalent substituent having a polymerizable carbon-carbon double bond copolymerizable with the polymerizable monomer, a cyano group, a C₁₋₆ alkyl group, a C₁₋₆ alkoxy group, a halogen atom and nitro group. Preferably, R¹ is a monovalent substituent having a polymerizable carbon-carbon double bond copolymerizable with the polymerizable monomer, a C₁₋₆ alkyl group or a halogen atom. More preferably R¹ is a monovalent substituent having a polymerizable carbon-carbon double bond copolymerizable with the polymerizable monomer or a halogen atom.

In a preferred embodiment, R¹ is a monovalent substituent having a polymerizable carbon-carbon double bond copolymerizable with the polymerizable monomer, wherein the polymerizable group is a vinyl group, an allyl group, a (meth)acryloyl group, a (meth)acrylamide group or an allyl (meth)acrylamide group, more preferably a vinyl group, a (meth)acryloyl group or an allyl (meth)acrylamide group, even more preferably a (meth)acryloyl group.

In formula (IIa) or (lib), n is 0 or an integer of from 1 to 4, preferably n is 0 or an integer of from 1 to 2, more preferably n is 0 or 1, even more preferably n is 0.

In formula (IIa) ot (lib), R² and R³ which may be the same or different, independently represent a saturated aliphatic hydrocarbon group having one to eight carbon atoms, an allyl group, or R² and R³ form together with the nitrogen atom to which they are bonded a C₃₋₆ saturated carbocyclic group which may contain a heteroatom selected from an oxygen atom and a sulfur atom, or R² and R³ form together with the nitrogen atom to which they are bonded a cyclic group further containing in the ring a group of the formula (III), preferably R² and R³ represent a saturated aliphatic hydrocarbon group having one to eight carbon atoms, or R² and R³ form together with the nitrogen atom to which they are bonded a cyclic group further containing in the ring a group of the formula (III), more preferably R² or R³ represent a saturated aliphatic hydrocarbon group having one to eight carbon atoms.

In a particularly preferred embodiment, R² and R³ which may be the same or different, independently represent a saturated aliphatic hydrocarbon group having one to eight carbon atoms, preferably a saturated aliphatic hydrocarbon group having one to four carbon atoms, more preferably one to two carbon atoms, and most preferably R² and R³ represent a methyl group.

In a specific embodiment, R² and R³ form together with the nitrogen atom to which they are bonded a C₃₋₆ saturated carbocyclic group which may contain a heteroatom selected from an oxygen atom and a sulfur atom, preferably a C₄ saturated carbocyclic group which may contain a heteroatom selected from an oxygen atom and a sulfur atom. Preferably, R² and R³ form together with the nitrogen atom to which they are bonded a C₃₋₆ saturated carbocyclic group which may contain a heteroatom selected from an oxygen atom, more preferably a C₄ saturated carbocyclic group which may contain a heteroatom selected from an oxygen atom. Particularly preferable, R² and R³ form together with the nitrogen atom to which they are bonded a moiety of the following formula:

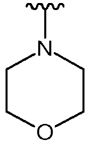

In a preferred embodiment, R² and R³ form together with the nitrogen atom to which they are bonded a cyclic group further containing in the ring a group of the formula (III), wherein Ar, R¹, n, X¹, X² and Q, independently are as defined above.

In formula (lib), X² and X¹ may be the same or different, and independently represent a single bond, -NR⁴- , -O-, or -S-, wherein R⁴ is a hydrogen atom, a C₁₋₆ alkylene group, a C₃₋₆ cycloalkylene group or an allyl group. Preferably, X² and X¹ independently represent a single bond, -NR⁴- or-O-, wherein R⁴ is a hydrogen atom, a C₁₋₆ alkylene group, a C₃₋₆ cycloalkylene group or an allyl group, more preferably X² and X¹ independently represent a single bond, -NR⁴- or -O-, wherein R⁴ is a hydrogen atom or an allyl group. Still more preferably X² and X¹ independently represent a single bond, -NR⁴- or -O-, wherein R⁴ is an allyl group. According to a specific embodiment, one of X² and X¹ is -O- and the other is a single bond or -NR⁴-. Preferably, X² and X¹ are not at the same time single bonds. Preferably, X² is a single bond.

The coinitiator (b-2) of formula (I) according to the present invention is a compound that contains at least one polymerizable carbon-carbon double bond copolymerizable with the polymerizable monomer and/or at least two groups selected from X and a group of formula (III).

In a preferred embodiment, the coinitiator (b-2) of formula (I) is a compound containing a polymerizable group having a carbon-carbon double bond. In another embodiment the coinitiator (b-2) of formula (I) is a compound containing at least two groups selected from X and a group of formula (III).

In a preferred embodiment, the coinitiator (b-2) of formula (I) is a compound having one or more polymerizable groups which are selected from a (meth)acrylate group and a (meth)acrylamide group.

In a preferred embodiment, the coinitiator (b-2) of formula (I) is a compound wherein R² and R³ which may be the same or different, independently represent a C₁₋₆ alkyl group.

In a preferred embodiment, the coinitiator (b-2) of formula (I) is a compound wherein R² and R³ represent a methyl group.

In a preferred embodiment, the coinitiator (b-2) of formula (I) is a compound wherein R² and R³ form together with the nitrogen atom to which they are bonded a cyclic group further containing in the ring a group of the formula (III) wherein Ar, R¹, n, X¹, X² and Q, in the group of formula (IIa) or (lib) and (III) are the same.

In a preferred embodiment, the coinitiator (b-2) of formula (I) is a compound wherein Ar is an o- or p-phenylene group.

Preferred classes of coinitiators (b-2) of formula (I) relate to compounds of the following formulae (I-A) to (I-H):

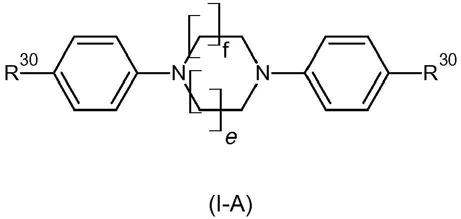

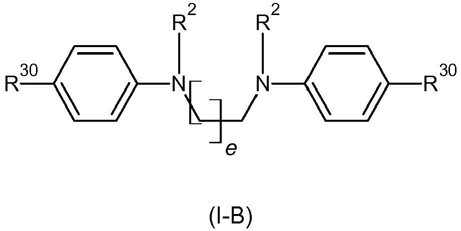

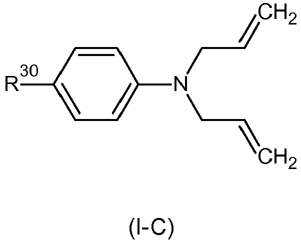

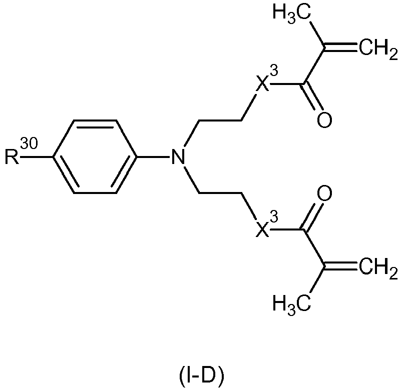

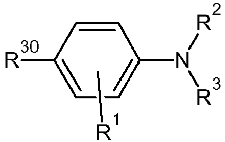

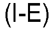

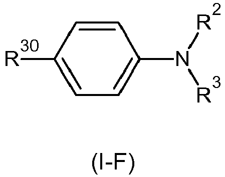

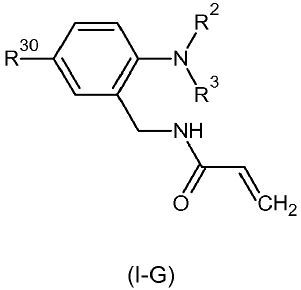

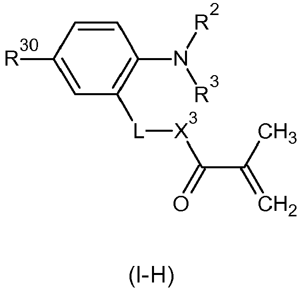

In formulae (I-A) to (I-H), R³⁰ is A-Q'-X¹C(O)X²-, wherein A, Q', X¹, and X² are as defined above; X³ is as defined for X¹/X²; R¹, R² and R³ are as defined above; L is an aliphatic hydrocarbon group or a polyoxyalkylene group, which have two to twelve carbon atoms and which may have up to two carbon-carbon double bonds copolymerizable with the polymerizable monomer; and e and f are independently 0 or integers of from 1 to 4.

Particularly preferred specific coinitiators (b-2) according to the present invention are compounds of the formulae:

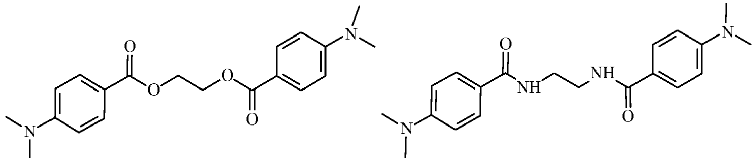

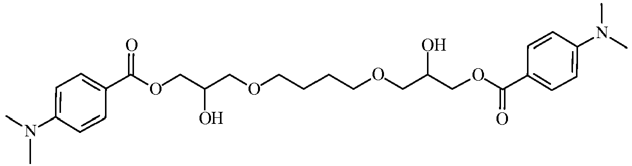

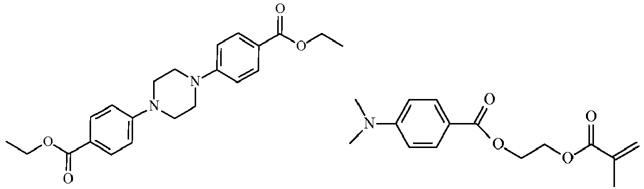

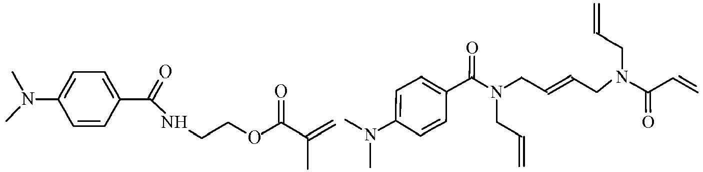

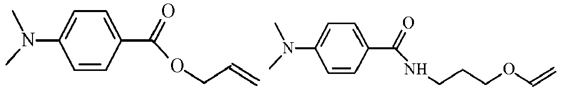

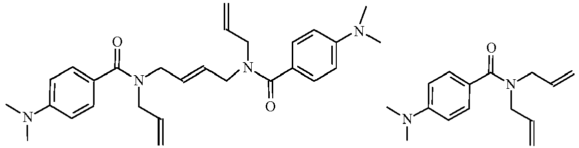

### Further optional components

The dental composition according to the present invention may, besides of the above described components, comprise additional optional components.

For example, the dental composition according to the present invention may comprise water, or any solvent known in the art.

The dental composition of the present invention may preferably comprise 5 to 20 percent by weight based on the total weight of the composition of water.

Optionally, the dental composition may further comprise stabilizer(s), and/or pigments.

The dental composition may further comprise one or more particulate filler.

The particulate filler(s) may be dental filler(s) known in the art. Preferably, the dental filler(s) are selected from particulate glass fillers and radiopaque fillers. More preferably, the dental filler(s) are selected from radiopaque filler(s).

The term "particulate glass filler" refers to a solid mixture of mainly metal oxides transformed by a thermal melt process into a glass and crushed by various processes. The glass is in particulate form. Moreover, the particulate glass filler may be surface modified, e.g. by silanation or acid treatment.

Preferably, the particulate glass filler is in spherical form.

For the dental fillers, a glass component may be selected from "inert glass(es)", "reactive glass(es)" and "fluoride releasing glass(es)".

The term "inert glass(es)" refers to a glass which is not capable of reacting with a polymer containing acidic groups in a cement reaction. Inert glasses are for example described in the Journal of Dental Research June 1979, pages 1607-1619, or more recently in US 4814362, US 5318929, US 5360770, and application US 2004/0079258 A1. Specifically, from US 2004/0079258 A1, inert glasses are known in which strongly basic oxides such as CaO, BaO, SrO, MgO, ZnO, Na₂O, K₂O, Li₂O etc. are replaced with weakly basic oxides such as those in the Scandium or Lanthanide series.

The term "reactive glass(es)" refers to a glass which is capable of reacting with a polymer containing acidic groups in a cement reaction. The glass is in particulate form. Any conventional reactive dental glass may be used for the purpose of the present invention. Specific examples of particulate reactive glasses are selected from calcium alumino silicate glass, calcium alumino fluorosilicate glass, calcium aluminumfluoroborosilicate glass, strontium aluminosilicate glass, strontium aluminofluorosilicate glass, strontium aluminofluoroborosilicate glass. Suitable reactive glasses may be in the form of metal oxides such as zinc oxide and/or magnesium oxide, and/or in the form of ion-leachable glasses, e.g., as described in US-A 3,655,605, US-A 3,814,717, US-A 4,143,018, US-A 4,209,434, US-A 4,360,605 and US-A 4,376,835.

The term "fluoride releasing glass(es)" refers to a glass capable to of releasing fluoride. Fluoride releasing capability may be provided by adding to a mixture of oxides for forming a glass inorganic particle(s) containing fluoride with the proviso that the glass has fluoride releasability, preferably sustained fluoride releasability. Such inorganic particles may be selected from the group consisting of sodium fluoride, strontium fluoride, lanthanum fluoride, ytterbium fluoride, yttrium fluoride, and calcium-containing fluoroaluminosilicate glasses.

Preferably, the particulate glass filler is a reactive glass or a fluoride releasing glass as defined above, more preferably a reactive glass.

More preferably, the particulate glass filler is a reactive particulate glass filler comprising:
1) 20 to 45% by weight of silica,
2) 20 to 40% by weight of alumina,
3) 20 to 40% by weight of strontium oxide,
4) 1 to 10% by weight of P₂O₅, and
5) 3 to 25% by weight of fluoride.

The present curable dental two-pack composition preferably comprises 20 to 90 percent by weight of the particulate glass filler, more preferably 30 to 80 percent by weight, based on the total weight of the composition.

The particulate glass filler usually has an average particle size of from 0.005 to 100 µm, preferably of from 0.01 to 40 µm, more preferably of from 0.05 to 20 µm, most preferably of from 0.1 to 3 µm as measured, for example, by electron microscopy or by using a conventional laser diffraction particle sizing method as embodied by a MALVERN Mastersizer S or MALVERN Mastersizer 3000 apparatus.

The particulate glass filler may have a unimodal or multimodal (e.g., bimodal) particle size distribution, wherein a multimodal particulate glass filler represents a mixture of two or more particulate fractions having different average particle sizes.

Preferably, the particulate filler is a radiopaque filler.

Suitable radiopaque particulate fillers may be selected from fillers containing elements of the group comprising tungsten, bismuth, strontium, barium, tantalum, cerium, tin, zirconium, ytterbium and yttrium.

The radiopaque particulate filler usually has an average particle size of from 0.005 to 100 µm, preferably of from 0.01 to 40 µm as measured using, for example, by electron microscopy or by using a conventional laser diffraction particle sizing method as embodied by a MALVERN Mastersizer S or MALVERN Mastersizer 2000 apparatus. The radiopaque particulate reactive glass may be a multimodal radiopaque particulate reactive glass representing a mixture of two or more radiopaque particulate fractions having different average particle sizes. The radiopaque particulate reactive glass may also be a mixture of particles of different chemical composition. In particular, it is possible to use a mixture of a radiopaque particulate reactive material and a radiopaque particulate non-reactive material.

The dental composition according to the invention preferably comprises 1 to 80 percent by weight, more preferably 40 to 70 percent by weight, of the radiopaque particulate filler, based on the weight of the entire composition.

In a specific embodiment, wherein the dental composition is a composition comprising a photoinitiator system and/or the dental composition is a dental impression material, the composition preferably may further comprise a particulate filler which has preferably a mean particle size in the range of from 0.05 to 5 µm as measured, for example, by electron microscopy or by using a conventional laser diffraction particle sizing method as embodied by a MALVERN Mastersizer S or MALVERN Mastersizer 2000 apparatus. The particulate filler may be a multimodal particulate filler representing a mixture of two or more particulate fractions having different average particle sizes. The particulate reactive filler may also be a mixture of particles of different chemical composition. The dental composition comprises 10 to 60 percent by weight, more preferably 20 to 50 percent by weight, based on the total weight of the dental composition of a filler. The specific type of filler is not particularly limited. Accordingly, any toxicologically acceptable inorganic, especially hydrophobic fillers may be employed such as silicas, aluminas, magnesias, titanias, inorganic salts, metallic oxides and glasses. The filler may be a mixtures of different fillers such as silicone dioxides including crystalline forms, in particular particulate quartz, amorphous silicon dioxides, in particular diatomaceous earth, and silanated fumed silica.

The viscosity and thixotropicity of the uncured as well as the physical properties of the cured compositions may be controlled by varying the sizes and surface areas of the filler.

The filler may be surface treated with one or more silanating agents. Preferred silanating agents include those having at least one polymerizable double bond and at least one group that easily hydrolyses with water. Examples of such agents include 3-methacryloxypropyltrimethoxysilane, 3-methacryloxypropyldimethoxy-monochlorosilane, 3-methacryloxypropyldichloromonomethoxysilane, methacryloxypropyltri-chlorosilane, 3-methacryloxypropyldichloromonomethyl-silane, 3- methacryloxypropylmonochlorodimethylsilane, and mixtures thereof.

Preferred filler are fumed silica, quartz, cristobalite, calcium silicate, diatomaceous earth, zirconium silicate, montmorillonite such as bentonite, zeolite, including molecular sieves such as sodium aluminium silicate, metal oxide powder such as aluminium or zinc oxide or their mixed oxides, barium sulphate, calcium carbonate, plaster, and glass powder.

### Examples

The present invention will now be further illustrated by the following examples.

### Example 1 - MAB 2-121-1

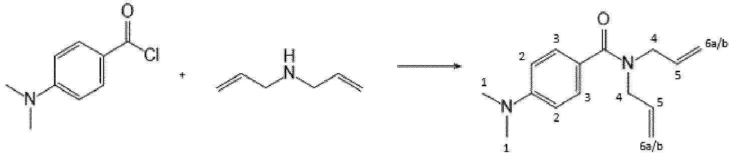

4-Dimethylaminobenzoyl chloride (10.00 g, 0.0545) was suspended in 25 ml of ethyl acetate and cooled down to 4 °C. Diallylamine (6.35 g, 0.065 moles) was dissolved in 25 ml ethyl acetate and triethylamine (5.51 g, 0.0545 moles) and was added to the acid chloride with stirring. After complete addition of latter, the reaction mixture was allowed to stir overnight at room temperature. After completion of the reaction, further 300 ml ethyl acetate was added to the reaction mixture and extracted with dilute sodium bicarbonate. The organic phase was dried with MgSO₄ and ethyl acetate was removed by rotary evaporation. The crude product was dispersed in MTBE, cooled in the refrigerator for 1h, filtered and finally washed with ice cooled ethanol. After drying 4.19 g were obtained (yield 32%).
¹H-NMR [ppm]: (300 MHz, CDCl₃): δ 7.40 (m, 2H, **H 3**), 6.66 (m, 2H, **H 2**), 5.82 (m, 2H, **H 5**), 5.20 - 5.18 (m, 4H, **H 6a/b**), 4.01 (s, 4H, **H 4**), 2.98 (s, 6H, **H 1)**

### Example 2 - MAB 2-124-1

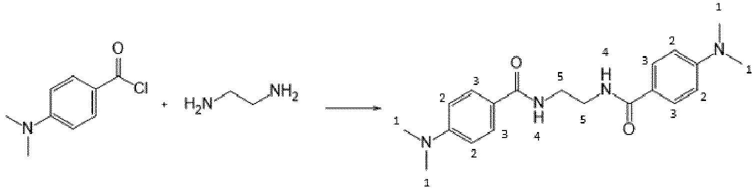

4-Dimethylaminobenzoyl chloride (10.00 g, 0.0545) was suspended in 25 ml of ethyl acetate. Ethlendiamin (1.47 g, 0.025 moles) was dissolved in 25 ml ethyl acetate and triethylamine (5.51 g, 0.0545 moles) and was added to the acid chloride with stirring. After complete addition of latter, the reaction mixture was allowed to stir overnight at room temperature. After completion of the reaction, the crude product was precipitated in ice cooled ethanol, filtered and finally vacuum dried. After drying 5.87 g were obtained (yield 31.5%).
¹H-NMR [ppm]: (300 MHz, CDCl₃): δ 7.74 (m, 2H, **H 3**), 7.06 (sb, 2H, **H 4**), 6.67 (m, 2H, **H 2**), 3.65 (t, 4H, **H 5**), 2.98 (m, 4H, **H 1**)

### Example 3 - MAB 2-127-1

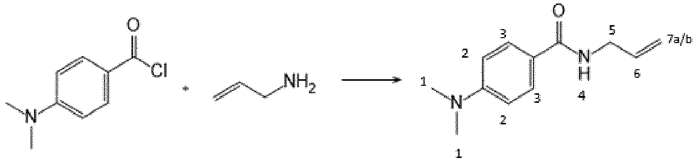

4-Dimethylaminobenzoyl chloride (10.00 g, 0.0545) was suspended in 25 ml of ethyl acetate. Allylamine (3.73 g, 0.065 moles) was dissolved in 25 ml ethyl acetate and triethylamine (5.51 g, 0.0545 moles) and was added to the acid chloride with stirring. After completion of the reaction, further 300 ml ethyl acetate was added to the reaction mixture and extracted with dilute sodium bicarbonate. The organic phase was dried with MgSO₄ and ethyl acetate was removed by rotary evaporation. The crude product was dispersed in ethanol, cooled in the refrigerator for 1h, filtered and finally washed with ice cooled ethanol. After drying 4.95 g were obtained (yield 44.5%).
¹H-NMR [ppm]: (300 MHz, CDCl₃): δ 7.69 (m, 2H, **H 3**), 6.64 (m, 2H, **H 2**), 6.16 (sb, 1H, **H 4**), 5.94 (m, 1H, **H 6**), 5.26 - 5.13 (m, 2H, **H 7a/b**), 4.06 (t, 2H, **H 5**), 3.00 (s, 6H, **H 1)**

### Example 4 - MS1580 (Comparative)

### Polym. tBDA

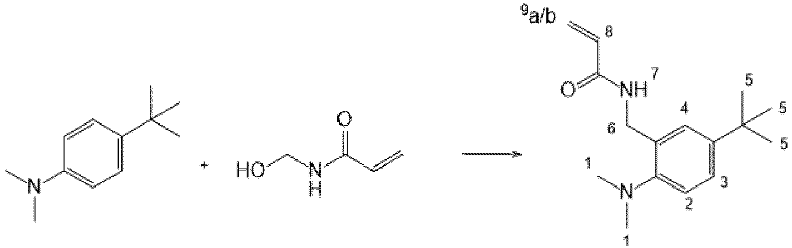

Aluminium chloride (2.26 g, 0.0169 mol) was given to a stirring solution of N-Hydroxyethyl acrylamide (1.71 g, 0.0169 mol) solved in 70 ml acetone in small portions. To the resulting white suspension, 4-tert-butyl-*N,N*-dimethylaniline (2.00 g, 0.0113 mol) solved in 10 ml acetone was given. The reaction mixture was refluxed for 24 h. After that, 70 ml of a 2N NaOH was given to the reaction mixture and the crude product was extracted twice with dichloromethane. The collected organic layer was dried by means of Na₂SO₄. The crude product was preliminary purified by means of column chromatography (ethyl acetate/2-Methylpentane 1:1). The oily residual was given to a freezer (-30 °C) which allows the crystallization of the product. Latter was divided from the oily residual and washed with 2-Methylpentane before drying.
¹H-NMR [ppm]: (300 MHz, CDCl₃): δ 7.29 (m, 2H, **H 2-3**), 7.1 (m, 1H, **H 4**), 7.04 (sb, 1H, **H 7**), 6.24 (m, 1H, **H 8**), 6.11 (m, 1H, **H 9a**), 5.61 (m, 1H, **H 9b**), 4.64 (d, 2H, **H 6**), 2.69 (s, 6H, **H 1**), 1.28 (s, 9H, **H 5**)

### Photo activated composition

To a mixture of Bis-GMA (70)/TGDMA (30) were added 0.5% CQ and 0.6% of an amine and mixed homogeneously. Composition and polymerization enthalpy measured with the DSC 7 (Perkin Elmer) is summarized in Table 1.

**Table 1: Composition and polymerization enthalpy of Photo activated Bis-GMA (70)/TGDMA (30) composition**

| Photo activated composition | Amine | ΔH_{R} [kJ/mol] |
|---|---|---|
| 1 | MAB 2-121-1 | -46.6 ± 2.2 |
| 2 | MAB 2-124-1 | -43.1 ± 3.2 |
| 3 | MAB 2-127-1 | -42.8 ± 0.4 |
| 4 (Ref.) | EDB* | -44.2 ± 0.9 |

| | | |
|---|---|---|
| *4-(dimethylamino) benzoic acid ethylester | | |

### PhotoDSC measurements

The polymerization enthalpies of the different formulations were measured at 37 °C using a photo DSC apparatus (DSC 7 from Perkin Elmer) equipped with a Xenon lamp (OSRAM XBO 450 W/1).

### Formulation of dental adhesives

### Abbreviations

Me2-DPI: Bis(4-methylphenyl)iodonium hexafluorophosphate
DMABN: 4-(Dimethylamino)benzonitrile
DMADA: 4-(dimethylamino)-*N*,*N*-di-2-propen-1-yl-benzamide
DMAEA: 4-Dimethylamino-*N*-[2-[(4-dimethylaminobenzoyl)amino]ethyl]benzamide

**Table 2: Formulation of Example 5 & 6 and Comparative Example 7**

| | **Example 5** | | **Example 6** | | **Comparative Example 7** | |
|---|---|---|---|---|---|---|
| | **[wt.-%]** | **[g]** | **[wt.-%]** | **[g]** | **[wt-%]** | **[g]** |
| Resin matrix | 61.9 | 2.48 | 61.9 | 2.48 | 61.9 | 2.48 |
| Solvent | 15.31 | 0.613 | 15.48 | 0.62 | 15.53 | 0.62 |
| Water | 19.33 | 0.77 | 19.49 | 0.78 | 19.54 | 0.78 |
| Camphorquinone | 1.55 | 0.062 | 1.55 | 0.062 | 1.55 | 0.062 |
| DMABN | - | - | - | - | 0.65 | 0.026 |
| Me2-DPI | 0.75 | 0.03 | 0.75 | 0.03 | 0.75 | 0.03 |
| DMADA | 1.074 | 0.043 | - | - | - | - |
| DMAEA | - | - | 0.75 | 0.03 | - | - |
| Inhibitor | 0.08 | 0.0032 | 0.08 | 0.0032 | 0.08 | 0.0032 |
| **SUM** | **100.00** | **4.00** | **100.00** | **4.00** | **100.00** | **4.00** |

### Preparation

Described amounts of components according to table 1 were given together in a light-tight glass container and stirred overnight.

### Testing: Shear bond strength (SBS)

The shear bond strength of Example 5 & 6 and Comparative Example 7 were measured according to DIN EN ISO 29022 using extracted human molars.

**Table 3: Results for the SBS on enamel and dentin (SE-mode)**

| **Sample** | **Example 5** | **Example 6** | **Comparative Example 7** |
|---|---|---|---|
| | **SBS [MPa] ± SD** | **SBS [MPa] ± SD** | **SBS [MPa] ± SD** |
| Enamel | 22 ± 2.3 | 22.7 ± 5 | 22 ± 5.2 |
| | (LAN 27-2-3) | (LAN 27-3-2) | (LAN 27-2-1) |
| Dentin | 34.2 ± 5.2 | 33.9 ± 5.3 | 35.2 ± 3.3 |
| | (LAN 27-2-1) | (LAN 27-3-3) | (LAN 27-2-2) |

### Formulation of dental RMGI's

### Abbreviations

tBDA: 4-*tert*-Butyl-N,N-dimethylaniline
MS 1580 - polym. tBDA: 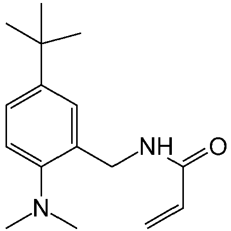
NapTS: Sodium-p-toluene sulfinate
KPS: Pottasium persulfate
CQ: Camphorquinone

**Table 4: Formulation of Example 8 and Comparative Example 9**

| | | ***Example 8*** | ***Comparative Example 9*** |
|---|---|---|---|
| | | **[wt%]** | ***[wt%]*** |
| **Liquid** | Water | 32.372 | 32.778 |
| | Cross-linker | 15.000 | 15.000 |
| | Modified polyacid | 25.655 | 25.655 |
| | Acrylic acid | 24.247 | 24.594 |
| | tBDA | 0 | 1.734 |
| | Poly. tBDA | 2.547 | 0 |
| | CQ | 0.179 | 0.179 |
| | Inhibtitor | 0.060 | 0.060 |
| | Σ | 100 | 100 |
| | | | |
| **Powder** | Reactive glass mixture | 99.139 | 99.139 |
| | NapTS | 0.661 | 0.661 |
| | KPS | 0.200 | 0.200 |
| | Σ | 100 | 100 |
| | | | |
| **P/L ratio** | | 3.3 | 3.3 |

### Preparation

Aqueous dental glass ionomer compositions of Example 8 according to the invention and of the Comparative Example 9 have been prepared by forming a liquid and a powder composition of the ingredients listed in Table 2, which respectively add up to 100 wt%, and admixing both parts in the shown powder/liquid (P/L) ratio.

### Testing

### [Flexural strength/ E-Modulus]

The obtained dental glass ionomer compositions of Example 8 and Comparative Example 9 were filled in a stainless-steel mold having the size (25 ± 2) mm x (2.0 ± 0.1) mm x (2.0 ± 0.1) mm, for the preparation of test specimens. The thus obtained dental glass ionomer compositions were cured with a dental curing light (light-cured, LC). For the resulting cured dental glass ionomer composition, the flexural strength has been determined according to ISO 9917-2.

### [Curing time]

Working time: Period of time, measured from the start of mixing the powder and glass in the shown P/L ratio, during which it is possible to manipulate the material without an adverse effect on the properties.

**Table 5: Results for working time, FS and E-modulus for example 8 and comparative example 9**

| | | ***Example 8*** | | ***Comparative Example 9*** | |
|---|---|---|---|---|---|
| Curing time | Working time (seconds) | 77 | VZY01-61-01 | 63 | VZY01-61-01 |
| Flexural strength (LC) [MPa] | | 116 ± 9 | PAP01-121-01 | 119 ± 6 | PAP01-119-01 |
| E-Modulus (LC) [MPa] | | 12420 ± 505 | PAP01-121-01 | 13525 ± 715 | PAP01-119-01 |

### Formulation of dental composites

### Abbreviations

Me2-DPI: Bis(4-methylphenyl)iodonium hexafluorophosphate
DMABE: 4-(Dimethylamino)benzonitrile
DMADA: 4-(dimethylamino)-*N*,*N*-di-2-propen-1-yl-benzamide
DMAEA: 4-Dimethylamino-*N*-[2-[(4-dimethylaminobenzoyl)amino]ethyl]benzamide

**Table 6: Liquid formulation of Example 10, 11 & 12 and Comparative Example 13**

| | **Example 10** (KJ 24-164-2) | | **Example 11** (KJ 24-165-2) | | **Example 12** (KJ 24-170-2) | | **Comparative Example 13** (KJ 24-166-2) | |
|---|---|---|---|---|---|---|---|---|
| | **[wt.-%]** | **[g]** | **[wt.-%]** | **[g]** | **[wt.-%]** | **[g]** | **[wt.-%]** | **[g]** |
| Resin matrix | 96.52 | 19.31 | 96.84 | 19.37 | 96.91 | 19.38 | 96.74 | 19.34 |
| Me2-DPI | 0.74 | 0.148 | 0.74 | 0.148 | 0.74 | 0.148 | 0.74 | 0.148 |
| Inhibitor | 0.75 | 0.150 | 0.75 | 0.150 | 0.75 | 0.150 | 0.75 | 0.150 |
| Pigment | 0.01 | 0.002 | 0.01 | 0.002 | 0.01 | 0.002 | 0.01 | 0.002 |
| UV-stabilizer | 0.60 | 0.120 | 0.60 | 0.120 | 0.60 | 0.120 | 0.60 | 0.120 |
| Camphorquinone | 0.24 | 0.048 | 0.24 | 0.048 | 0.24 | 0.048 | 0.24 | 0.048 |
| DMABE | - | - | - | - | | | 0.9 | 0.180 |
| DMADA | 1.14 | 0.228 | - | - | | | - | - |
| DMAEA | - | - | 0.82 | 0.164 | | | - | - |
| Q1 | | | | | 0.75 | 0.155 | | |
| **SUM** | **100.00** | **20.00** | **100.00** | **20.00** | **100.00** | **20.00** | **100.00** | **4.00** |

### Preparation

The liquid systems were finally mixed with glass composition in the ratio 22:78.

Described amounts of components according to table 1 were put together in a light-tight plastic container. Each container was subsequently placed in the SpeedMixer DAC 600-2 VAC-P (Hauschild) and mixed twice at 2500 rpm for 2 min and once at 1000 rpm/100 mbar for 1 min. After that, the composite was prepared by mixing 22 wt% of the liquid composition according to table 1 with 78 wt% of a composite glass filler composition and mixed again twice at 2500 rpm for 2 min and once at 1000 rpm/100 mbar for 1 min in the SpeedMixer.

### Testing

The flexural strength (FS), Depth of Cure (DoC) and Sensitivity to ambient light were measured according to the procedures described in the ISO 4049:2009 guideline.

**Table 7: Results for DoE and STAI for example 10, 11 & 12 and comparative example 13**

| **Method** | **Flexural strength [MPa]** | **DoC [mm]** | **STAL [sec]** |
|---|---|---|---|
| Example 10 | 140 ± 9 (KJ 24-169-1) | 5.9 (KJ 24-167-1) | 40 (KJ 24-168-2) |
| Example 11 | 131 ± 9 (KJ 24-169-1) | 3.0 (KJ 24-167-2) | 180 (KJ 24-168-2) |
| Example 12 | 135 ± 9 (KJ 24-171-1) | 3.7 (KJ 24-172-2) | 170 (KJ 24-173-2) |
| Comparative Example 13 | 125 ± 19 (KJ 24-169-1) | 5.3 (KJ 24-168-1) | 100 (KJ 24-168-2) |

## Claims

1. Use of a coinitiator of the following formula (I):
Q-X (I)
wherein
Q is a cyano group or a group A-Q'-;
wherein
A is a hydrogen atom, a further group X, or a polymerizable group having a carbon-carbon double bond copolymerizable with the polymerizable monomer;
Q' is an aliphatic hydrocarbon group or a polyoxyalkylene group, which have two to twelve carbon atoms and which may have up to two carbon-carbon double bonds and/or polymerizable groups having a carbon-carbon double bond, which are copolymerizable with the polymerizable monomer, and which may be substituted with a group selected from a hydroxy group;
X which may be the same or different when more than one X is present, independently represents a group of the following formula (IIa) when Q is a cyano group or a group of the following formula (IIb) when Q is a group A-Q'-:
-Ar(R¹)ₙ-NR²R³ (IIa)
-X¹C(O)X²Ar(R¹)ₙ-NR²R³ (IIb)
wherein
X¹ and X² which may be the same or different, independently represent a single bond, -NR⁴- , -O-, or -S-, wherein R⁴ is a hydrogen atom, a C₁₋₆ alkylene group, a C₃₋₆ cycloalkylene group or an allyl group;
Ar is an (n+2)-valent aromatic hydrocarbon group;
R¹ which may be the same or different if more than one R¹ is present, independently represents a monovalent substituent having a polymerizable carbon-carbon double bond copolymerizable with the polymerizable monomer, a cyano group, a C₁₋₆ alkyl group, a C₁₋₆ alkoxy group, a halogen atom or a nitro group;
n is 0 or an integer of from 1 to 4;
R² and R³, which may be the same or different, independently represent a saturated aliphatic hydrocarbon group having one to eight carbon atoms, an allyl group, or R² and R³ form together with the nitrogen atom to which they are bonded a C₃₋₆ saturated carbocyclic group which may contain a heteroatom selected from an oxygen atom and a sulfur atom, or R² and R³ form together with the nitrogen atom to which they are bonded a cyclic group further containing in the ring a group of the following formula (III)
>NAr(R¹)ₙX²C(O)X¹Q, (III)
wherein Ar, R¹, n, X¹, X² and Q, independently are as defined above;
provided that the compound of formula (I) contains at least one polymerizable carbon-carbon double bond copolymerizable with the polymerizable monomer and/or at least two groups selected from X and a group of formula (III),
for the preparation of a dental composition selected from a dental bonding agent, dental sealer, a dental composite, a resin-modified dental cement, and a dental impression material, comprising
(a) a polymerizable monomer; and
(b) an initiator system comprising
(b-1) an alpha diketone sensitizer absorbing light within a range of about 400 nm to about 520 nm or an oxidizing agent of a redox initiator system; and
(b-2) the coinitiator.

2. The use according to claim 1, wherein the coinitiator of the formula (I) is a compound containing a polymerizable group having a carbon-carbon double bond.

3. The use according to claim 1, wherein the coinitiator of the formula (I) is a compound containing at least two groups selected from X and a group of formula (III).

4. The use according to any one of the preceding claims wherein the polymerizable group is selected from a (meth)acrylate group and a (meth)acrylamide group.

5. The use according to any one of claims 1 to 4, wherein Q is a cyano group and X is a group of the formula (IIa).

6. The use according to any one of claims 1 to 4, wherein Q is a group A-Q'- and X is a group of the formula (IIb).

7. The use according to any one of the preceding claims, wherein R² and R³, which may be the same or different, independently represent a C₁₋₆ alkyl group.

8. The use according to any one of claims 1 to 6, wherein R² and R³ form together with the nitrogen atom to which they are bonded a C₃₋₆ saturated carbocyclic group containing a heteroatom selected from an oxygen atom and a sulfur atom.

9. The use according to any one of claims 1 to 4, wherein R² and R³ form together with the nitrogen atom to which they are bonded a cyclic group further containing in the ring a group of the formula (III) wherein Ar, R¹, n, X¹, X² and Q, in the group of formula (IIa) or (IIb), and (III) are the same.

10. The use according to any one of the preceding claims, wherein Ar is an o- or p-phenylene group.

11. The dental composition according to any one of the preceding claims, wherein the sensitizer is camphor quinone.

12. The use according to any one of the preceding claims, further comprising a solvent and/or a particulate filler.

## Patentansprüche

1. Nutzung eines Coinitiators der folgenden Formel (I):
Q-x (I)
wobei
Q eine Cyanogruppe oder eine Gruppe A-Q'- ist;
wobei
A ein Wasserstoffatom, eine weitere Gruppe X oder eine polymerisierbare Gruppe mit einer mit dem polymerisierbaren Monomer copolymerisierbaren Kohlenstoff-Kohlenstoff-Doppelbindung ist;
Q' eine aliphatische Kohlenwasserstoffgruppe oder eine Polyoxyalkylengruppe mit zwei bis zwölf Kohlenstoffatomen ist, die bis zu zwei Kohlenstoff-Kohlenstoff-Doppelbindungen und/oder polymerisierbare Gruppen mit einer Kohlenstoff-Kohlenstoff-Doppelbindung aufweisen kann, die mit dem polymerisierbaren Monomer copolymerisierbar sind und die mit einer Gruppe substituiert sein kann, die aus einer Hydroxygruppe ausgewählt ist;
X , das gleich oder verschieden sein kann, wenn mehr als ein X vorhanden ist, unabhängig für eine Gruppe der folgenden Formel (IIa) steht, wenn Q eine Cyanogruppe ist, oder eine Gruppe der folgenden Formel (IIb), wenn Q eine Gruppe A-Q'- ist:
-Ar(R¹)ₙ-NR²R³ (IIa)
-X¹C(O)X²Ar(R¹)ₙ-NR²R³ (IIb)
wobei
X¹ und X², die gleich oder verschieden sein können, unabhängig voneinander eine Einfachbindung, -NR⁴-, -O- oder -S- darstellen, wobei R⁴ ein Wasserstoffatom, eine C₁₋₆-Alkylengruppe, eine C₃₋₆- Cycloalkylengruppe oder eine Allylgruppe ist;
Ar eine (n+2)-wertige aromatische Kohlenwasserstoffgruppe ist;
R¹ , das gleich oder verschieden sein kann, wenn mehr als ein R¹ vorhanden ist, unabhängig voneinander einen einwertigen Substituenten mit einer polymerisierbaren Kohlenstoff-Kohlenstoff-Doppelbindung darstellt, die mit dem polymerisierbaren Monomer, einer Cyanogruppe, einer C₁₋₆-Alkylgruppe, einer C₁₋₆-Alkoxygruppe, einem Halogenatom oder einer Nitrogruppe copolymerisierbar ist;
n gleich 0 oder eine ganze Zahl von 1 bis 4 ist;
R² und R³, die gleich oder verschieden sein können, unabhängig voneinander eine gesättigte aliphatische Kohlenwasserstoffgruppe mit 1 bis 8 Kohlenstoffatomen darstellen, eine Allylgruppe, oder R² und R³ zusammen mit dem Stickstoffatom, an das sie gebunden sind, eine C₃₋₆-gesättigte carbocyclische Gruppe bilden, die ein Heteroatom, ausgewählt aus einem Sauerstoffatom und einem Schwefelatom, enthalten kann, oder R² und R³ zusammen mit dem Stickstoffatom, an das sie gebunden sind, eine cyclische Gruppe bilden, die ferner im Ring eine Gruppe der folgenden Formel (III) enthält
> NAr(R¹)ₙX²C(O)X²Q, (III)
wobei Ar, R¹, n, X¹, X² und Q unabhängig wie oben definiert sind;
vorausgesetzt, dass die Verbindung der Formel (I) zumindest eine polymerisierbare Kohlenstoff-Kohlenstoff-Doppelbindung enthält, die mit dem polymerisierbaren Monomer und/oder zumindest zwei Gruppen, ausgewählt aus X und einer Gruppe der Formel (III), copolymerisierbar ist, für die Herstellung einer Dentalzusammensetzung, ausgewählt aus einem Dentalbindemittel, einem Dentalversiegeler, einem Dentalkomposit, einem harzmodifizierten Dentalzement und einem Dentalabformmaterial, umfassend
(a) ein polymerisierbares Monomer; und
(b) ein Initiatorsystem, umfassend
(b-1) einen Alpha-Diketon-Sensibilisator, der Licht innerhalb eines Bereichs von etwa 400 nm bis etwa 520 nm absorbiert, oder ein Oxidationsmittel eines Redox-Initiatorsystems; und
(b-2) den Coinitiator.

2. Nutzung nach Anspruch 1, wobei der Coinitiator der Formel (I) eine Verbindung ist, die eine polymerisierbare Gruppe mit einer Kohlenstoff-Kohlenstoff-Doppelbindung enthält.

3. Nutzung nach Anspruch 1, wobei der Coinitiator der Formel (I) eine Verbindung ist, die zumindest zwei Gruppen enthält, die aus X und einer Gruppe der Formel (III) ausgewählt sind.

4. Nutzung nach einem der vorhergehenden Ansprüche, wobei die polymerisierbare Gruppe aus einer (Meth)acrylatgruppe und einer (Meth)acrylamidgruppe ausgewählt ist.

5. Nutzung nach einem der Ansprüche 1 bis 4, wobei Q eine Cyanogruppe ist und X eine Gruppe der Formel (IIa) ist.

6. Nutzung nach einem der Ansprüche 1 bis 4, wobei Q eine Gruppe A-Q'- und X eine Gruppe der Formel (IIb) ist.

7. Nutzung nach einem der vorhergehenden Ansprüche, wobei R² und R³, die gleich oder unterschiedlich sein können, unabhängig eine ₁₋₆-Alkyl-Gruppe darstellen.

8. Nutzung nach einem der Ansprüche 1 bis 6, wobei R² und R³ zusammen mit dem Stickstoffatom, an das sie gebunden sind, eine gesättigte carbocyclische C₃₋₆-Gruppe bilden, die ein Heteroatom enthält, das aus einem Sauerstoffatom und einem Schwefelatom ausgewählt ist.

9. Nutzung nach einem der Ansprüche 1 bis 4, wobei R² und R³ zusammen mit dem Stickstoffatom, an das sie gebunden sind, eine cyclische Gruppe bilden, die im Ring ferner eine Gruppe der Formel (III) enthält, worin Ar, R¹, n, X¹, X² und Q in der Gruppe der Formel (IIa) oder (IIb) und (III) gleich sind.

10. Nutzung nach einem der vorhergehenden Ansprüche, wobei Ar eine o- oder eine p-Phenylengruppe ist.

11. Dentalzusammensetzung nach einem der vorhergehenden Ansprüche, wobei der Sensibilisator Kampfer-Chinon ist.

12. Dentalzusammensetzung nach einem der vorhergehenden Ansprüche, ferner umfassend ein Lösungsmittel und/oder einen partikulären Füllstoff.

## Revendications

1. Utilisation d'un co-amorceur de formule (I) suivante :
Q-x (I)
où
Q est un groupe cyano ou un groupe A-Q'- ;
dans lequel
A est un atome d'hydrogène, un autre groupe X, ou un groupe polymérisable ayant une double liaison carbone-carbone copolymérisable avec le monomère polymérisable ;
Q' est un groupe hydrocarboné aliphatique ou un groupe polyoxyalkylène, qui a deux à douze atomes de carbone et qui peut avoir jusqu'à deux doubles liaisons carbone-carbone et/ou des groupes polymérisables ayant une double liaison carbone-carbone, qui sont copolymérisables avec le monomère polymérisable, et qui peuvent être substitués par un groupe choisi parmi un groupe hydroxy ;
X qui peut être identique ou différent lorsque plus d'un X est présent, représente indépendamment un groupe de formule (IIa) suivante lorsque Q est un groupe cyano ou un groupe de formule (IIb) suivante lorsque Q est un groupe A-Q'- :
-Ar (R¹)ₙ-NR²R³ (IIa)
-X¹C(O)X²Ar(R¹)ₙ-NR²R³ (IIb)
où
X¹ et X² qui peuvent être identiques ou différents, représentent indépendamment une liaison simple,-NR⁴-, -O- ou -S-, où R⁴ est un atome d'hydrogène, un groupe alkylène en C₁₋₆, un groupe cycloalkylène en C₃₋₆ ou un groupe allyle ;
Ar est un groupe hydrocarboné aromatique de valence (n+2) ;
R¹ qui peut être identique ou différent si plus d'un R¹ est présent, représente indépendamment un substituant monovalent ayant une double liaison carbone-carbone polymérisable copolymérisable avec le monomère polymérisable, un groupe cyano, un groupe alkyle en C₁₋₆, un groupe alcoxy en C₁₋₆, un atome d'halogène ou un groupe nitro ;
n vaut 0 ou est un entier de 1 à 4 ;
R² et R³ , qui peuvent être identiques ou différents, représentent indépendamment un groupe hydrocarboné aliphatique saturé ayant un à huit atomes de carbone, un groupe allyle, ou R² et R³ forment conjointement avec l'atome d'azote auquel ils sont liés un groupe carbocyclique saturé en C₃₋₆ qui peut contenir un hétéroatome choisi parmi un atome d'oxygène et un atome de soufre, ou R² et R³ forment conjointement avec l'atome d'azote auquel ils sont liés un groupe cyclique contenant en outre dans le cycle un groupe de formule (III) suivante
>NAr(R¹)ₙX²C(O)X²Q, (III)
où Ar, R¹, n, X¹, X² et Q, indépendamment, sont tels que définis ci-dessus ;
à condition que le composé de formule (I) contienne au moins une double liaison carbone-carbone polymérisable copolymérisable avec le monomère polymérisable et/ou au moins deux groupes choisis parmi X et un groupe de formule (III),
pour la préparation d'une composition dentaire choisie parmi un agent de liaison dentaire, un agent de scellement dentaire, un composite dentaire, un ciment dentaire modifié par résine et un matériau d'empreinte dentaire, comprenant
(a) un monomère polymérisable ; et
(b) un système amorceur comprenant
(b-1) un sensibilisateur à l'alpha-dicétone absorbant la lumière dans une plage d'environ 400 nm à environ 520 nm ou un agent oxydant d'un système amorceur d'oxydoréduction ; et
(b-2) le co-amorceur.

2. Utilisation selon la revendication 1, dans laquelle le co-amorceur de formule (I) est un composé contenant un groupe polymérisable ayant une double liaison carbone-carbone.

3. Utilisation selon la revendication 1, dans laquelle le co-amorceur de formule (I) est un composé contenant au moins deux groupes choisis parmi X et un groupe de formule (III).

4. Utilisation selon l'une quelconque des revendications précédentes, dans laquelle le groupe polymérisable est choisi parmi un groupe (méth)acrylate et un groupe (méth)acrylamide.

5. Utilisation selon l'une quelconque des revendications 1 à 4, dans laquelle Q est un groupe cyano et X est un groupe de formule (IIa).

6. Utilisation selon l'une quelconque des revendications 1 à 4, dans laquelle Q est un groupe A-Q'- et X est un groupe de formule (IIb).

7. Utilisation selon l'une quelconque des revendications précédentes, dans laquelle R² et R³, qui peuvent être identiques ou différents, représentent indépendamment un groupe alkyle en C₁₋₆.

8. Utilisation selon l'une quelconque des revendications 1 à 6, dans laquelle R² et R³ forment conjointement avec l'atome d'azote auquel ils sont liés un groupe carbocyclique saturé en C₃₋₆ contenant un hétéroatome choisi parmi un atome d'oxygène et un atome de soufre.

9. Utilisation selon l'une quelconque des revendications 1 à 4, dans laquelle R² et R³ forment conjointement avec l'atome d'azote auquel ils sont liés un groupe cyclique contenant en outre dans le cycle un groupe de formule (III) où Ar, R¹, n, X¹, X² et Q, dans le groupe de formule (IIa) ou (IIb), et (III) sont identiques.

10. Utilisation selon l'une quelconque des revendications précédentes, dans laquelle Ar est un ou groupe o- ou p-phénylène.

11. Composition dentaire selon l'une quelconque des revendications précédentes, dans laquelle le sensibilisateur est la camphorquinone.

12. Utilisation selon l'une quelconque des revendications précédentes, comprenant en outre un solvant et/ou une charge particulaire.
